# EUROPEAN PATENT SPECIFICATION

(11) **EP 0 134 242 B1**
(45) Date of publication and mention of the grant of the patent: **09.06.1993**
(21) Application number: 84900931.1
(22) Date of filing: 27.01.1984
(51) Int. Cl.: C12P 21/00, C12N 15/30, A61K 39/00, C07K 15/02

(54) **PROTECTIVE PEPTIDE ANTIGEN**
SCHÜTZENDES PEPTID-ANTIGEN
ANTIGENE DE PEPTIDE PROTECTEUR

(30) Priority: 28.01.1983 GB 8302349
(43) Date of publication of application: 20.03.1985
(73) Proprietor: NEW YORK UNIVERSITY, New York, NY 10003 (US)
(72) Inventor: COLMAN, David, R., New York, NY 10003 (US); ELLIS, Joan, New York, NY 10022 (US); GODSON, G., Nigel, New York, NY 10012 (US); NUSSENZWEIG, Ruth, S., New York, New York 10012 (US); NUSSENZWEIG, Victor, N., New York, NY 10012 (US); SCHLESINGER, David, H., Plainsboro, NJ 08536 (US); SVEC, Pamela, S., New York, NY 10012 (US); ZAVALA, Fidel, New York, NY 10028 (US)
(74) Representative: Grünecker, Kinkeldey, Stockmair & Schwanhäusser Anwaltssozietät
(86) International application number: US8400144
(87) International publication number: WO8402922

(56) References cited:
- WO-A-84/02917
- CHEMICAL ABSTRACTS, vol. 97, no. 9, 30 August 1982, page 152, abstract 67168g, Columbus, Ohio, US; M. GOMAN et al.: "The establishment of genomic DNA libraries for the human malaria parasite Plasmodium falciparum and identification of individual clones by hybridization" & MOL. BIOCHEM. PARASITOL. 1982, 5(6), 391-400
- NATURE, vol. 302, (Letters to Nature), 7 April 1983, pages 536-538, Chesham, Bucks, GB; J. ELLIS et al.: "Cloning and expression in E.coli of the malarial sporozoite surface antigen gene from Plasmodium knowlesi"
- NATURE, vol. 305, no. 5929, September 1983, pages 29-33, Chesham, Bucks, GB; G.N. GODSON et al.: "Identification and chemical synthesis of a tandemly repeated immunogenic region of Plasmodium knowlesi circumsporozoite protein"
- BIOLOGICAL ABSTRACTS/RRM, no. 26058722, Bioscience Information Service, Philadelphia, PA, US; J. SCAIFE et al.: "Application of recombinant DNA techniques to the human malaria parasite Plasmodium falciparum", 12th Annual UCLA Symposium on Molecular Biology of Host-Parasite Interactions, Journal of Cellular Biochem. Suppl, 1983, vol. 0, no. 7A, page 3
- CHEMICAL ABSTRACTS, vol. 98, no. 23, 6 June 1983, page 170, abstract 192681r, Columbus, Ohio, US; N. BONE et al.: "Investigation of the DNA of the human malaria parasite Plasmodium falciparum by in vitro cloning into phage lambda", & SERONO SYMP. PUBL. RAVEN PRESS 1983, 2(Mol. Biol. Parasites), 125-34
- Yoshida et al., J. Exp. Med., Vol. 154, pp. 1225-1236, Oct. 1981
- Nardin et al., J. Exp. Med., Vol. 156, pp. 20-30, July 1982
- Cochrane et al., P.N.A.S., USA, vol. 79, pp. 5651-5655, Sept. 1982
- Yoshida et al., Science, Vol. 207, pp. 71-73, January 4, 1980
- Kilejian, Am. J. Trop. Med. Hyg. 29(5) Suppl., pp. 1125-1128, 1980

## Description

The government has rights in the invention based upon research support in the form of Grant No. 5R01-Ail7429-03 from the Department of Health and Human Services and Grant No. AID-DPE-0453-C-00-2002-00 from the Department of State, Agency for International Development.

This application is a continuation-in-part of copending commonly assigned U.S. Patent Application Serial No. 234,096 of Nussenzweig R.S., Nussenzweig V. and Godson N.G., filed February 12, 1981, now allowed.

### BACKGROUND OF THE INVENTION

The present invention relates to the field of antigens suitable for providing protective immunity against malaria when incorporated into a vaccine. Malaria constitutes a worldwide public health hazard of enormous economic and medical significance. The disease contributes substantially to infant mortality in endemic areas and remains a severe and debilitating illness for those who remain afflicted with it as adults. Despite advances in the techniques of mosquito abatement and improved public health measures, regions where the disease is considered endemic are increasing in area. Furthermore, the risk of infection has substantially increased in some parts of the world because of the occurrence of new drug-resistant strains of the malaria parasite.

The causative agent of malaria is a protozoan of the genus Plasmodium. Individual species within the genus appear to have a restricted host range for the animals they infect. For example, P.berghei and P.yoeli are infective to rodents, P.knowlesi and P.cynomolgi are primarily infective to monkeys, while P.falciparum, P.vivax, P.ovale and P. malariae are the species primarily infective to humans. Despite species differences in host range, the life cycles, mode of infection, biochemistry and genetics of the various Plasmodium species are markedly similar.

The life cycle of Plasmodium is complex, the organism undergoing several distinct morphological changes, involving the participation of a mammalian host and a mosquito vector. The parasite, in the sporozoite form, is introduced to the mammalian host through the bite of the mosquito vector. The sporozoites rapidly disappear from the blood stream and are next found as intracellular parasites of liver parenchymal cells. A blood infection ensues, characterized by the well-known clinical symptoms of malaria after a complex series of morphological and biochemical transitions. The parasite is then found in the red blood cells, where it continues its development. Substantial amounts of the parasite may be obtained from the red blood cells of infected patients.

Vaccine development, to provide protective immunity against malaria infection has been thwarted by the fact that the parasite's life cycle in the mammalian host is primarily intracellular. Except for brief periods of time, the parasite is protected from contact with the immune system. Two stages in the parasite's life cycle during which it becomes briefly exposed to the immune system are, 1) the interval following initial infection before sporozoites have successfully invaded the cells of the liver and 2) the interval during which merozoites leave infected red blood cells and enter uninfected red blood cells. The transient exposure of the merozoite forms in the extracellular milieu has provided the basis for prior art attempts to develop host immunity to blood forms of the parasite. European published Patent Application, Number 62924, discloses antigenic proteins useful in the making of a vaccine to provide immunity against merozoite forms of the parasite. The utility of such a vaccine would presumably lie in limiting or arresting the course of the established malaria infection.

An alternative approach, based upon sporozoite antigens has led to the discovery of antigenic and immunogenic proteins of sporozoites that are capable of providing protective immunity against initial infection, when administered as a vaccine, Cochrane, A. H., et al., in Malaria, vol. 3, (J. Kreier, ed.) Academic Press N.Y. (1980) pp. 163-202; Nussenzweig, R. S. in Immunity to Blood Parasites of Animals and Man, (L. Miller, J. Pino and J. McKelvey, eds.) Plenum, N.Y. (1977) pp. 75-87. Gwadz, R. W., et al., Bull, W. H. O. Suppl. 1, 57, 165 (1979); Clyde, D. F., et al., Am. J. Trop, Med. and Hyg. 24, 397 (1975); McCarthy, V., et al., Exp. Parasitol. 41, 167 (1977).

These proteins were shown to be immuno reactive with antibodies raised against all sporozoites. Nardin et al. (J. Exp.Med. 156, pages 20 to 30, 1982) could determine the molecular weight of the immuno reactive Plasmodium falciparum. Nardin et al. could determine the molecular weight of the immuno reactive Plasmodium falciparum proteins to be 67 and 58kD, whereas immuno reactive Plasmodium vivax proteins were of 45 and 51kD. Similarly, Cochrane et al. (Proc. Natl. Acad. Sci. USA 79, pages 5651 to 5655, 1982) could show the immuno reactive protein of Plasmodium knowlesi to exhibit a molecular weight between 44 and 52kD.

These immunogenic proteins are antigenically distinguishable for each Plasmodium species, but have numerous structural properties in common including chromatographic behavior, isoelectric point, and electrophoretic mobility. The sporozoite antigens range in molecular weight from approximately 40,000 daltons to 70,000 daltons and have low isoelectric points, Santoro, F. et al., J. Biol. Chem. 258, 3341, 1983.

The comparison of tryptic digests of purified sporozoite antigen proteins of different Plasmodium species shows that several tryptic peptides have identical retention times on reverse-phase high performance liquid chromatography, indicating that there is a high degree of homology between antigenic proteins of different species.

The sporozoite antigens are components of the sporozoite surface coat. The presence of the sporozoite antigens is indicated by a characteristic immunologic reaction known as the circumsporozoite reaction, and by immunofluorescence tests. See Vanderberg, J. P., et al, Mil. Med. (Suppl.) 134, 1183 (1969); and Nardin, E., et al, Nature 274, 55 (1978).

These reactions make it possible to specifically detect the sporozoite antigen for a given Plasmodium species, without resorting to time-consuming in vivo tests. This, in turn, has made it possible to develop specific radioimmunoassays for sporozoite antigens, and ultimately for the production of malaria antibodies directed against sporozoite antigens of Plasmodium species.

Antibodies against the sporozoite antigens have been shown to provide protective immunity against the Plasmodium species from which they were derived, in rodents, monkeys and in human volunteers. The sporozoite protective antigen protein is herein termed CS protein, circumsporozoite protein, or sporozoite CS protein, these terms being deemed equivalent. A co-pending U.S. application, Serial No. 234,096, filed February 12, 1981, has been filed, disclosing a vaccine based upon purified CS protein. Said application (a copy of which is annexed hereto as Appendix A) is incorporated herein by reference as though set forth in full.

The results disclosed herein are based in part on the techniques and concepts of the field of immunology. For convenience, certain terms commonly used in the art are defined herein. The term "immunochemical reaction" is used to denote the specific interaction which occurs between an antigen and its corresponding antibody, regardless of the method of measurement. Such a reaction is characterized by a non-covalent binding of one or more antibody molecules to one or more antigen molecules. The immunochemical reaction may be detected by a large variety of immunoassays known in the art. The terms "immunogenic" or "antigenic" will be used here to describe the capacity of a given substance to stimulate the production of antibodies specifically immunoreactive to that substance when that substance is administered to a suitable test animal under conditions known to elicit antibody production. The term "protective antigen" refers to the ability of a given immunogen to confer resistance in a suitable host, against a given pathogen. The term "epitope", refers to a specific antibody binding site on an antigen. Macromolecular antigens such as proteins typically have several epitopes with distinctive antibody binding specificities. Different epitopes of the same antigen are distinguishable with the aid of monoclonal antibodies which, due to their high degree of specificity, are directed against single epitopes. Two different monoclonal antibodies directed against different epitopes on the same antigen may each bind the antigen without interfering with the other, unless the epitopes are so close together that the binding of one sterically inhibits the binding of the other. The term "immunodominant region" denotes an area of the antigen molecule which is mainly responsible for its antigenicity.

It was an object of the present invention to provide a possibility allowing the mass production of peptides eliciting antibodies recognizing the native sporozoite CS protein. This object is achieved by providing a peptide containing at least one occurrence of an amino acid sequence corresponding to a repeating unit of the immunodominant epitope region of the circumsporozoite protein of a member of the genus Plasmodium, said region containing tandem occurences of said unit and said peptide comprising all or part of said region, but not being the entire circumsporozoite protein, wherein said unit contains up to twelve amino acids and said peptide elicits species specific antibodies recognizing the circumsporozoite protein upon injection of said peptide into a mammal.

### SUMMARY OF THE INVENTION

The present invention is based on the discovery that the protective CS sporozoite antigens of the genus Plasmodium possess a single immunodominant region composed of repetitions of the same epitope. For P.knowlesi, the epitope has been shown to be a dodecapeptide whose sequence is repeated several times within the structure of the CS protein. The repeated peptide has been chemically synthesized in both monomeric and dimeric forms. The synthetic repeated peptide is immunochemically reactive with polyclonal antibody preparations against P.knowlesi. In addition, all monoclonal antibodies against CS proteins which neutralize the infectivity of sporozoites in vitro, also react with the synthetic peptide. Therefore, the synthetic repeated peptide constitutes substantially all of the immunogenic activity displayed by the naturally occurring sporozoite protective antigen of P.knowlesi.

Several lines of evidence indicate that CS proteins of the Plasmodium species infective to rodents, monkeys and humans are structurally similar. All possess an immunodominant region composed of similarly repeated epitopes. For each species, the repeated peptide of the sporozoite CS protein can be synthesized. The repeated peptide of a CS protein is immunogenic when administered in a composition, and by administration methods, known in the art to yield antibody production. On the basis of the discoveries and teachings herein described, structural determination and synthesis of the repeated peptide corresponding to any Plasmodium species sporozoite, and the preparation of a vaccine composition incorporating said peptide and capable of eliciting protective immunity against said species is now available to those of ordinary skill in the art.

As further confirmation of the close relationship of CS proteins of different Plasmodium species, monoclonal antibodies against P.knowlesi sporozoites have been shown to cross-react with P.falciparum antigen, a species infective to humans. It is therefore apparent that the development of other synthetic peptides more specifically reactive with human malaria species are well within the grasp of those ordinarily skilled in the art, following the teachings and disclosures as set forth herein.

### DETAILED DESCRIPTION OF THE INVENTION

In the following description, the materials employed were commercially available, unless otherwise specified. Enzymes used in the cloning procedures were obtained from commercial sources. Restriction endonuclease reactions were carried out according to the manufacturer's instructions. Unless otherwise specified, the reaction conditions for other enzyme reactions were standard conditions used in the art, as described, for example, in Methods in Enzymology, volume 60 (R. Wu, Ed.) Academic Press, (1980). Unless otherwise specified, the abbreviations herein are standard abbreviations acceptable for publication in scientific journals normally used by those skilled in the art to publish their results, such as those cited herein.

In general outline, the experiments and conclusions following from the results thereof are set forth. The approach taken herein to clone a DNA segment coding for the sporozoite antigen protein was to clone cDNA made from mRNA obtained from infected mosquitoes. The cDNA approach was preferred in the initial cloning work because it was not known whether Plasmodium genomic DNA contained introns that might prevent the expression of antigenically identifiable sporozoite proteins. Now that the short and repeated nature of the epitope is known, it is feasible to select for DNA encoding the epitopes from a library of genomic Plasmodium DNA. The initial experiments were performed with cDNA from mRNA of infected mosquitoes, since it was only at that stage that the Plasmodium was known to express the sporozoite antigen. A cDNA library was constructed from Poly (A)⁺RNA derived from P.knowlesi-infected mosquitoes. Double stranded cDNA, tailed with poly-C residues, were inserted into the plasmid pBR322, previously cut with Pst I and tailed with poly-G. Host cells transformed to tetracycline resistance were selected and single colonies of transformed cells were stored in microtiter dishes at -70°C.

The cDNA clones were screened for the ability to express a protein that contained the immunochemically reactive region of the sporozoite surface antigen. Once a cDNA coding for the sporozoite antigen was identified, others could readily be detected by hybridization using the originally cloned cDNA as a probe. Clones derived either from cDNA or genomic DNA libraries could be identified in this manner, based upon the homology between DNA segments coding for the sporozoite antigen of different Plasmodium species. Identification of clones expressing an immunoreactive protein was done by screening lysates of colonies of cells transformed (as above) with the cloned cDNA. Pools of 48 colonies were screened using a sensitive, two-site immunoradiometric assay performed with monoclonal antibodies. This permitted the detection of the CS protein in the transformed cells.

In short, a monoclonal antibody to P.knowlesi CS protein was adsorbed to the wells of a microtiter plate. Lysates from pools of 48 colonies were each added to the wells and incubated for sufficient time to allow the immunoreactive protein present in the lysate to bind to the adsorbed monoclonal antibody. The wells were then washed to remove any contaminating protein and a radio-labelled second monoclonal antibody to P.knowlesi CS protein was added. The labelled second monoclonal antibody attaches to the antigenic protein that is already bound to the surface of the microtitre well by the first monoclonal antibody. If a pool of 48 colonies was found to be positive, the colonies were screened individually in the same fashion. In this manner, positive clones were identified.

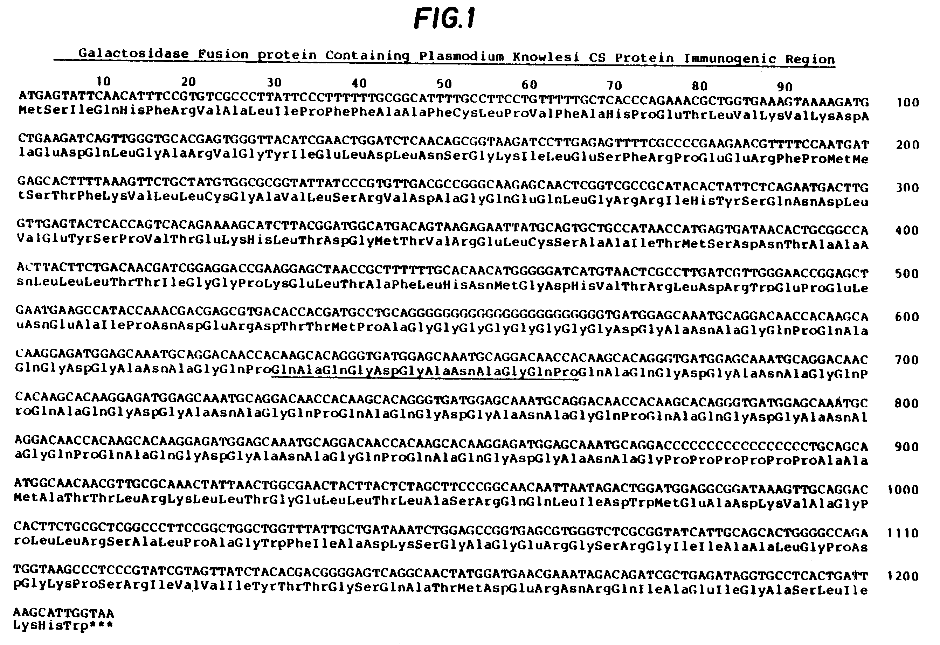

Whenever an immunoreactive clone was detected, plasmid DNA was isolated from it and used to transform another host cell strain, such as E.coli HB 101 or E.coli RR1. Transformants detected by tetracycline resistance were rechecked for the ability to express the immunochemically reactive protein, in order to confirm that the expression was a property of the plasmid DNA clone containing CS nucleotide sequences. Once suitable plasmid DNA was obtained from the positive clones, the nucleotide sequence of the cDNA insert coding for at least the immunoreactive region of the CS protein was obtained (by cloning onto M13). Methods of nucleotide sequence analysis are well known in the art, including the method of Maxam and Gilbert, W. Proc. Nat. Acad. Sci. USA 74, 560 (1977) and the method of Sanger, F. et al, Proc. Nat. Acad. Sci. USA 74, 5463 (1977). The latter method was employed in the present work. The complete nucleotide sequence of a segment of the P.knowlesi CS protein gene that contains the immunochemically reactive site is shown in Figure 1.

A surprising feature of the nucleotide sequence was that it was repetitive. In P. knowlesi the sequence consisted of a 36 base pair repeat, 8 complete units of which were represented in one clone (24-mer) together with partial units at either end. In order to deduce the amino acid sequence coded by the DNA, it was necessary to identify the coding strand and, within the strand, the correct reading frame. In this context, the advantage of using the Sanger and Coulson, supra sequencing method becomes evident. A sequencing vector, bacteriophage M13mp9, contains a betagalactosidase gene with a Pst I site in the same reading frame as the Pst I cleavage site of pBR322. Therefore, the reading frame can be deduced once the number of deoxy C-residues added during the tailing reaction is known and the sequencing vector can express a beta-galactosidase fusion protein comprising the immuno-chemically reactive part of the CS protein. Therefore, two different M13mp9 recombinants were obtained, with the 368 bp P.knowlesi DNA fragment inserted in opposite orientations. Only one of the two recombinants produced immuno-chemically reactive betagalactosidase fusion protein, as measured by the above-described radioimmunoassays. The clone producing the immunoreactive protein was used to identify the coding strand and direction of transcription of the P.knowlesi gene fragment.

The correct reading frame was also deduced using immunological procedures. These showed that the epitope defined by the monoclonal antibodies was destroyed by elastase, but not by trypsin nor by reducing agents, indicating that the epitope did not contain any of lysine, arginine or disulfide bonds, but might contain alanine residues.

On the basis of such experiments, the amino acid sequence of the 12 amino acid-containing repetitive peptide was deduced to be:
H₂N-GlnAlaGlnGlyAspGlyAlaAsnAlaGlyGlnPro-COOH.

To confirm the deduced amino acid sequence and immunochemical reactivity of the above-described sequence, a dodecapeptide of the same amino acid sequence and a dimer thereof were synthesized using an automated solid phase peptide synthesis system.

The monomer and dimer synthetic peptides were separately tested for immunochemical activity in the same type of radioimmunoassay as was used initially to screen the cDNA library. In this test, two antibody binding sites must be present in the antigen, one for binding to the first monoclonal attached to the microtiter well and the second for binding the added labelled antibody. Although the monomer peptide did not bind the labeled antibody, the dimer peptide was reactive, indicating that the dimer contained two complete, or nearly complete, antibody binding sites. Furthermore, the same assay showed that the monomer was able to compete with and specifically inhibit the binding of CS proteins of P.knowlesi to the microtiter wells bearing the first monoclonal antibody. Therefore, the sequence shown above contains an epitope of the sporozoite antigen. Another important observation was that all monoclonal antibodies to P.knowlesi obtained to date, as well as all polyclonal antibodies obtained from monkeys immunized with irradiated sporozoites, also reacted with the synthetic peptides. Actually, more than 70% of the antibodies to sporozoites, found in the serum of the immunized monkeys, recognized this single epitope (Zavala, et al., J. Exp. Med. 157:1947, 1983).

Therefore a chemically synthesized dodecapeptide having an amino acid sequence identical to that repeated in a sporozoite membrane protein contains substantially all of the antigenicity of the naturally occurring CS protein. It follows that based upon the principles of immunology and following techniques and procedures known to those of ordinary skill in the art, a synthetic peptide based upon the known amino acid sequence of a sporozoite CS protein can be incorporated into a vaccine composition capable of providing protective immunity in a host organism susceptible to a Plasmodium species from which the sequence of the peptide was derived. The following experiments, generally described, demonstrate the essential structural and functional similarities between the sporozoite CS proteins of the Plasmodium species infective to rodents, monkeys and humans. These similarities are exploitable to identify and synthesize the antigenic peptides specific for any Plasmodium species including, in particular, those infective to humans. The structural determination and synthesis of the repeated peptide for any Plasmodium species can be carried out by methods described herein or by equivalent methods known in the art, or by methods known in the art which exploit the disclosures and teachings of the present invention to eliminate some of the more time-consuming and tedious aspects of the original experiments. Of significance is that cross reactivity has been observed between monoclonal antibodies to the CS proteins of different Plasmodium species. For example, antibodies to the CS protein of P.knowlesi cross react with the CS protein of P.cynomolgi and P.falciparum; antibodies to the CS protein of P.cynomolgi cross react with the sporozoite antigen of P.vivax; antibodies to the CS protein of P.yoeli nigeriensis cross react with sporozoite antigen of P.berghei, and in that instance, completely neutralize the infectivity of sporozoites of the latter species, for mice.

Additional immunochemical evidence has been adduced to demonstrate that all sporozoite CS proteins have a single immunodominant region and repetitive epitopes Zavala, et al., supra. The binding of several different monoclonal antibodies directed against the same sporozoite CS protein was tested, measuring the inhibitory effects that the binding of one might have on the other. Monoclonal antibodies directed against different sequences within an antigen should not interfere with their respective binding capacities. Conversely, if monoclonals are directed against the same epitope, or epitopes which are topographically close, they inhibit each other. In the case of P.knowlesi, every one of the six monoclonals used, strongly inhibited the binding of the others to the antigen.

The same experiments were performed using monoclonals to the sporozoite antigen proteins of P.vivax, P.falciparum, P.malariae P.cynomolgi and P.berghei, with identical results. Therefore, these sporozoite CS proteins all are characterized by having a single immunodominant region.

Of direct relevance to the development of a vaccine against human malaria is the observation that antibodies in the serum of humans vaccinated and protected against sporozoites of P.falciparum or P.vivax are also directed against the same immunodominant region of the sporozoite CS protein. Pretreatment of a crude extract of sporozoites of either species with a single monoclonal antibody directed against the sporozoite antigen of the same species almost completely inhibited the subsequent binding of the antigen (in the sporozoite extract) to polyclonal antibodies isolated from the serum of the vaccinated human volunteers (Zavala, et al., supra).

The fact that immunodominant regions of the CS proteins contain repetitive epitopes was demonstrated by a solid phase two site radioimmunoassay. In the assay, a monoclonal antibody was bound to the plastic surface a microtiter well, antigen was added and the antigen was found to the immobilized antibody. The well was then washed to remove any unbound material and a second monoclonal antibody, presumably directed against a different epitope of the same antigen, was added. The second antibody is labelled with a radioisotope to quantitate the binding of the second antibody. Binding of the second antibody is proportional to the amount of antigen bound in the well. This immunoassay can be performed only if the antigen contains at least two epitopes. The first epitope binds to the antibody immobilized to the plastic of the plate, the second binds the radiolabelled antibody. Surprisingly, in the case of every CS protein, the two site radioimmunoassay could be performed using a single monoclonal antibody. That is to say, the assay could be performed using unlabelled monoclonal antibody A as the first monoclonal and the same monoclonal A as the second monoclonal. The result demonstrates that the sporozoite CS protein has at least two identical epitopes.

A control experiment demonstrated that the result was not an artifact caused by aggregation of the sporozoite antigen protein. Extracts of P.knowlesi sporozoites were dissolved in 2.0% (w/v) sodium dodecylsulfate and 6M urea and fractionated by ultracentrifugation in sucrose gradients. The existence of two epitopes was demonstrated in fractions of the gradient containing proteins of molecular weight 40,000, corresponding to the size of a CS protein monomer. Furthermore, there was no indication of the presence of aggregates of CS proteins. Identical results were obtained in experiments performed with P.vivax and P.falciparum extracts (Zavala, et al, supra).

It is therefore clear that all sporozoite CS proteins have a single immunodominant region comprising a peptide repeated many times within the protein. The repeated peptide contains the epitope, and each sporozoite CS protein is composed of a plurality of such repeated peptide epitopes. These epitopes are very immunogenic in all animal species, including man. Synthetic peptides containing the epitope of a given sporozoite CS protein are functionally identical to naturally occurring sporozoite antigens, with the obvious exception of two site radioimmunoassays requiring two epitopes on the same molecule. The functional behavior in two site assays is reproduced by synthetic dimers of the repeated peptide.

It will be readily appreciated therefore that synthetic peptides, comprising an amino acid sequence corresponding to an epitope of a sporozoite CS protein in monomeric or multimeric form, can be incorporated into vaccines capable of inducing protective immunity against sporozoites of malaria parasites, e.g., P.falciparum, P.vivax and P. malariae. Techniques for enhancing the antigenicity of such repeated peptides include incorporation into a multimeric structure, binding to a highly immunogenic protein carrier, for example, keyhole limpet hemocyanin, or diptheria toxoid, and administration in combination with adjuvants or any other enhancers of immune response. Furthermore, it will be understood that peptides specific for a plurality of Plasmodium stages and species may be incorporated in the same vaccine composition to provide a multivalent vaccine. In addition, the vaccine composition may comprise antigens to provide immunity against other diseases in addition to malaria.

An amino acid sequence corresponding to an epitope of a CS protein (repeated peptide) may be obtained by chemical synthetic means or by purification from biological sources including genetically modified microorganisms or their culture media. The repeated peptide may be combined in an amino acid sequence with other peptides including fragments of other proteins, as for example, when synthesized as a fusion protein, or linked to other antigenic or non-antigenic peptides of synthetic or biological origin. The term "corresponding to an epitope of a CS protein" will be understood to include the practical possibility that, in some instances, amino acid sequence variations of a naturally occurring repeated peptide may be antigenic and confer protective immunity against malaria sporozoite infection. Possible sequence variations include, without limitation, amino acid substitutions, extensions, deletions, interpolations and combinations thereof. Such variations fall within the contemplated scope of the invention provided the peptide containing them is antigenic and antibodies elicited by such peptide cross-react with naturally occurring CS protein or non-variant repeated peptides of CS protein, to an extent sufficient to provide protective immunity when administered as a vaccine. Such vaccine compositions will be combined with a physiologically acceptable medium. Routes of administration, antigen dose, number and frequency of injections are all matters of optimization within the scope of ordinary skill in the art, particularly in view of the fact that there is experience in the art in providing protective immunity by the injection of inactivated sporozoites. It is anticipated that the principal value of providing immunity to sporozoite infection will be for those individuals who have had no previous exposure to malaria, e.g., infants and children who live in endemic and subendemic areas, and unexposed adults travelling into endemic areas. It is also anticipated that temporary immunity for infants may be provided by immunization of mothers during pregnancy. Details of the operation and practice of the present invention are set forth in the specific examples which follow.

### EXAMPLE 1

### cDNA clone coding for a sporozoite antigen protein.

The techniques of recombinant DNA technology make extensive use of enzyme-catalyzed reactions. Purified enzymes for use in the practice of the present invention are currently available from commercial sources. Commercially available enzymes and reagents were employed unless otherwise specified. Restriction endonucleases, their nomenclature and site specificities, have been described in detail by Roberts, R.J., Nucl. Acids Res., 8, p. 63 (1980). The restriction enzymes used in this work were used in amounts and under reaction conditions specified by the manufacturer for each enzyme.

Approximately 1000 P.knowlesi infected mosquitoes grown, maintained and collected as described by Cochrane et al., Proc. Natl. Acad. Sci. USA 79:5651-5655 (1982) were harvested and dissected to obtain thoracic segments which were stored on ice until the dissection was completed. RNA was prepared from the thoraxes essentially as described by Seeburg, P.H., et al; Cell, 12, 157 (1977), and by Chirgwin, J. M., et al; Biochemistry, 24, 5294 (1979). The tissue was homogenized in 10ml of 5M guanidine thiocyanate, pH 5.0, 10mM EDTA and 0.1M 2-mercaptoethanol until all the tissue was dispersed. The solution was centrifuged at 10,000 rpm for 10 minutes and the supernatant adjusted to 2% (w/v) Sarkosyl (Trademark, ICN Pharmaceuticals, Plain-view, New York), and heated at 65°C for two minutes. Cesium chloride was then added (0.1g/ml of solution) and the resulting solution was layered over 2ml cushions of half-saturated CsCl in 10mM EDTA in SW41 (Trademark, Beckman Instruments, Fullerton, Calif.) cellulose nitrate tubes. Centrifugation was at 28,000 rpm for approximately 20 hours at 20°C. The RNA pellet was dissolved in 5mM EDTA, 0.5% (w/v) Sarkosyl and 5% (w/v) 2-mercaptoethanol, extracted with phenol and chloroform and precipitated with ethanol. Usually, 0.5-1 mg of RNA were obtained per g of tissue. The RNA was then passed over an oligo (dT)-cellulose column (Aviv, H., et al, Proc. Nat. Acad. Sci. USA, 69, 1408 (1972), to enrich for the polyadenylated fraction. Alternatively, the RNA can be prepared from the mosquito thoraces by the procedure modified from Liu, C.P., et al., Proc. Nat. Acad. Sci. 76:4503, 1979. According to this procedure, tissue was homogenized in 8-10 vol. of 4M guanidine isothiocyanate pH 5.0 (with glacial acetic acid) and 0.1 M 2-mercaptoethanol until the tissue was dispersed. Centrifugation took place at 9,000 rpm for 3 minutes, and the supernatant was layered over 0.2 vol. of 5.7M CsCl in 0.10M EDTA (pH 6.5) in SW 41 cellulose nitrate tubes. Centrifugation was at 35,000 rpm for 16-20 hours @ 20°C. Approximately 0.5-1 mg of RNA was obtained per gram of tissue. Poly(A)+RNA was then oligo-dT selected as described above.

A sample of mRNA isolated as described was translated in vitro using a translation system prepared from wheat germ (modified from Roberts, B.E., et al, Proc. Nat. Acad. Sci. USA, 70, 2330 (1973)). Proteins produced by in vitro translation were immunoprecipitated as described in Example 2, (alternatively, as disclosed by Goldman, B.M., and Blobel, G., Proc. Natl. Acad. Sci., 75:5066 (1978)) and fractionated on an SDS-polyacrylamide gel (SDS-Page) as described by Yoshida, et al, J. Exp. Med 154, 1225 (1981) and in Example 2 of copending U.S. application Ser. No. 234,096, incorporated herein by reference. mRNA fractions containing sequences coding for the CS-proteins can be identified by this means.

For preparative cDNA synthesis total polyadenylated mRNA (approximately 20 µg) was treated in 100 µl volume with 1mM methyl mercury (hereinafter MeHg) (Aldrich Chemical, Milwaukee, Wisconsin) at room temperature for 5 minutes. The treatment was stopped by adding 0.5% (0.5 µl per 100 µl) of undiluted β-mercaptoethanol and incubating at room temperature for 5 minutes. The MeHg treated polyadenylated mRNA was incubated in 200 ul reaction containing 50mM Tris-HCl pH 8.3, 10mM MgCl₂, 20mM KCl, 5mM Dithiothreitol, 2mM each of dATP, dCTP, dGTP and dTTP, 50 µCi ³²PdCTP (specific activity, 800 Ci/mmol), 4 µg oligo(dT)12-18, (Collaborative Research, Waltham, Massachusetts) 5 µl RNasin (BIOTECH, Madison, Wisconsin) and approximately 200 units reverse transcriptase (from Beard, Life Sci., St. Petersburg, Fla.). Incubation was at 42° for 60 minutes.

The reaction was stopped by extraction with phenol and chloroform (1:1), then with an equal volume of chloroform and precipitation by ethanol. The ethanol precipitate was dissolved in 50 µl 10mM Tris-HCl, 1mM EDTA, pH=8 and fractionated on a column of Sephadex (Trademark, Pharmacia, Inc., Uppsala, Sweden) G-75 in a 1ml Falcon (Trademark, Falcon Plastics, Oxnard, California) plastic pipette, using 10mM Tris-HCl, pH 7.4, and 1mM EDTA as the running buffer. The leading peak of unexcluded ³²P counts was collected (approximately 300 µl) and adjusted to 0.3M NaOH and 1mM EDTA and incubated overnight at room temperature. Following neutralization with 5M sodium acetate pH 3.8 to a final pH of approximately 6.0, and ethanol precipitation, the second DNA strand was synthesized in a 50 µl reaction containing the same buffer as described supra, 500 µM each of dATP, dCTP, dGTP, dTTP, 125 µCi ³²PdCTP (800Ci/mmole) and 50 units reverse transcriptase. Incubation was at 37°C for 90 minutes. The reaction products were extracted with phenol/chloroform and passed over a Sephadex G-75 column as described above and the excluded ³²P peak was precipitated with ethanol before proceeding to treatment with S₁ nuclease.

After fractionation on Sephadex G-75, the second cDNA strand synthesis was completed using the Klenow fragment of DNA polymerase I (Boehringer-Mannheim) in the presence of 50mM Tris-HCl, pH 8.0, 7mm MgCl₂ and 1mM dithiothreitol. The reaction mixture was incubated for 4 hours at 15°C, extracted with phenol-chloroform (1:1) and precipitated with ethanol as described above.

The double stranded cDNA was incubated with 300 units of Sl nuclease (Boehringer-Mannheim, Indianapolis, Indiana) in 24 µl of 0.3mM NaCl, 30mM Ma acetate, pH 4.5, and 3mM ZnCl₂ at 41°C for 5 minutes. The reaction was stopped by the addition of EDTA to 10mM and neutralized with Tris base. The ³²P-labelled cDNA was size fractionated using a column of Sepharose CL-4B (Trademark, Pharmacia, Inc., Uppsala, Sweden) made up in a lml Falcon plastic pipette and run in a buffer of 0.3M NaCl, 10mM Tris-HCl pH 8.0, lmM EDTA. Various size classes of double-stranded cDNA were precipitated with ethanol and then tailed using calf thymus terminal transferase (Enzo. Biochem., Inc., New York, New York) in a 100 µl volume for 1 minute at 37°C in a buffer containing 100mM K cacodylate, pH 7.6, lmM CoCl₂, 0.1mM DTT, 0.1mM dCTP, 4 µCi of ³H-dCTP, 24Ci/mmole), and approximately 20 units terminal transferase/µm ds-cDNA. The reaction was stopped by adjusting the solution to O.5M NaCl, 10mM EDTA and incubating at 65°C for 5 min. 1-5 micrograms of yeast + RNA were added prior to extraction with phenol: chloroform and precipitated with ethanol twice. The tailing reaction is described, generally, by Roychoudhury, R., et al, Nucl. Acids Res., 3, 101 (1976). An alternative procedure, also conducted, is described by Land, H., et al, Nucl. Acids. Res. 9:2251, 1981. Approximately 17 bases were added to the 3' end under these conditions. Plasmid pBR322 cleaved by Pst I endonuclease and tailed with dG residues was obtained from a commercial source, New England Nuclear, Boston, Massachusetts. Equimolar amounts of dC tailed cDNA and dG tailed pBR322 were annealed at a concentration of 1 µg/ml, using sequential 2 hour incubations at 42°C, 30°C, and 14°C. The hybrid plasmid DNA was ethanol-precipitated and then used to transform E.coli RR1 cells to ampicillin resistance. Libraries of single colonies were generated and stored in microtiter dishes at -70°C.

According to the alternative procedure, after 15-30 deoxy C residues have been added to the cDNA and annealing to an equimolar concentration of dG-tailed pBR 322 has taken place (at 250ng of vector/ml), the annealing mixture was incubated at 68°C for 5 min, then at 42°C for two hours, followed by slow cooling to room temperature for 2 hours. The hybrid plasmid DNA was used to transform E.coli RRl cells to tet-resistance as described by Dagert, M. et al, Gene 6:23, 1979. A library was generated and stored as individual colonies in Luria broth with 15% glycerol in microtiter dishes at -70°C.

A library of (300-2000bp) cDNA fragments was screened for colonies that expressed protein containing the immunochemically reactive region of the sporozoite surface antigen protein. Forty-eight colonies were grown individually on a petri dish containing S agar (32g/litre tryptone, 5g/litre NaCl, 20g/litre yeast extract, 15g/litre Difco agar 0.2g/litre NaOH and 20mg/liter tetraclycline). The plates were flooded with 2mls of 0.05M Tris-HCl pH 7.5 and 0.5mg egg-white lysozyme (Sigma, St. Louis, Mo.) and scraped with a sterile spatula into a 15ml polypropylene tube (Fisher Scientific Supply). After incubation at room temperature for 30 minutes, followed by 60 minutes on ice freeze thawing in 95% ethanol and dry ice (-80°C) three times, and further incubation at 37°C for 10 minutes the crude cell extracts were treated with DNAse I (1mg/ml), 4mM CaCl₂ and 4mM MgCl₂ at room temperature for 30 minutes and stored at -70°C for future use.

The lysates were screened for the presence of any immunochemically reactive protein using P.knowlesi monoclonal antibodies, in a radioimmunoassay. In this method, anti-P.knowlesi monoclonal antibody adsorbed to the well of a microtiter dish was used to affinity purify any immunoreactive protein present in pooled cell lysate. Lysates containing an immunoreactive protein were detected by reacting the washed microtiter wells with a second ¹²⁵I-labelled anti-P.knowlesi monoclonal antibody. To do this, microtiter plates were coated with 50 µl anti P.knowlesi monoclonal antibody (50 µg/ml) incubated at 4°C for 12-17 hours, washed thoroughly with 1% (w/v) BSA-saline solution, and then incubated with 50 µl of the pooled cell extract for 4-17 hours at 4°C. After washing, a second ¹²⁵I-labelled anti-P.knowlesi monoclonal antibody was added to each well and incubated 2 hours at room temperature. The washed wells were then tested individually for radioactivity. When a pool of 48 colonies was found to be positive, the original single colonies that made up the pool were screened individually and the immunoreactive clones identified, isolated and genetically purified.

Plasmid DNA was purified from 1 litre of cells containing an immunoreactive clone (plasmid pEG81). The cells were grown at 37° in Luria broth with 15 µg/ml tetracycline to approximately 5X10⁸/cells per ml and the plasmid DNA amplified by adding 175 µg/ml of chloramphenicol and incubating overnight (Clewell and Helinski, J. Bacteriol. 110, 1135 (1972)). The plasmid DNA was extracted from the cells using sodium dodecyl sulphate (SDS) (Godson and Vapnek, Biochim. Biophys. Acta 299, 516 (1973)) and purified using 5-20% (w/v) sucrose density gradients. This yielded 500-1000 µg plasmid RF I DNA. 1 µg of this was used to transform other E.coli cells (HB101) to tetracycline resistance and their ability to express the immunoreactive protein was re-checked.

pEG81 DNA was digested with Pst I restriction endonuclease ligated with T4 DNA ligase to 0.5 µg of Pst I-cut cloning/sequencing vector M13mp9 at a 1:1 molar ratio and sequenced using the Sanger dideoxy chain termination method (Sanger and Coulson, supra) with a "universal" synthetic primer 5' - d[GTAAAACGACGGCCAGT] - 3' (purchased from PL Biochemicals, Milwaukee, Wisc.). The complete nucleotide sequence of this segment of the P.knowlesi CS protein gene that contains the immunoreactive site is shown in Figure 1.

An unexpected feature of the pEG81 fragment of P.knowlesi DNA is that it consisted entirely of a 36 base pair repeat (8 complete units plus a partial unit on either end). The coding strand and correct reading frame of the nucleotide sequence was established as follows:
(a) The reading frame of the Pst I cleavage site of pBR322 ampicillinase gene and of the M13mp9 β-galactosidase genes are known to be identical (5' X C T G C A G X X 3').
   Met Thr Met Ile Thr Pro Ser Leu Ala Ala Gly M13mp9 ATG ACC ATG ATT ACG CCA AGC TTG GCT GCA GGT
   Pst I Cleaving Site
   Two different M13mp9 recombinants were obtained with the P.knowlesi DNA fragment inserted in opposite orientations. One of the recombinants produced an immunoreactive β-galactosidase fusion protein (M13mp9/Pk 11) as measured by the radioimmunoassay described supra, the other clone did not. The sequence of M13mp9/Pk 11 therefore identified the coding strand of the DNA.
(b) The reading frame was also deduced from the fact that 17 dC residues were inserted between the β-gal gene and the P.knowlesi gene fragment.
(c) One possible reading frame coded for an alanine-rich peptide. That the epitope probably contained alanine was verified by treating authentic P.knowlesi CS surface protein with porcine elastase, known to cleave peptides at alanine residues (Powers, J. C., et al, Biochem, Biophys. Acta, 485: 156-166, (1977). Incubation of an extract of 10⁶ sporozoites with 0.002 units porcine elastase (Worthington Enzymes, infra) in Tris-buffer, 0.05M pH 8.6, for 60 minutes at 37°C, completely abrogated the reactivity of the CS protein with monoclonal antibodies as determined by a two-site immunoradiometric assay described in Example 4. Inactivation of the CS protein by elastase was reversed by the synthetic inhibitor OOC-Ala Ala Pro Ala (Powers, et al supra).

Other possible reading frames coded for peptides which were excluded for various reasons: they were too hydrophobic or they contained several cysteine, lysine and arginine residues. The absence of such amino acid residues had been determined in experiments showing that the epitope was resistant to trypsin after complete reduction of the CS protein. Indeed, after incubation of another aliquot of the same sporozoite extract with lmg of TPCK-trypsin (Worthington Enzymes, Freehold, New Jersey) at 37°C for 30 minutes, followed by complete reduction and alkylation, the CS protein reacted fully with the monoclonal antibodies.

The deduced amino acid sequence of the twelve amino acid repeat is as follows based upon translation of the nucleotide sequence in the correct reading frame:
Gln-Ala-Gln-Gly-Asp-Gly-Ala-Asn-Ala-Gly-Gln-Pro
(All sequences are expressed from the end nearest NH₂ - terminus on the left to the end nearest the -COOH terminus on the right.) The immunoreactive portion of the P.knowlesi protein is therefore contained within the 12 amino acid repeat.

To confirm that the foregoing amino acid sequence contains the immunoreactive site, a dodecapeptide (with the same order of amino acids as shown above) and a dimer of the dodecapeptide have been synthesized, using solid phase resin synthesis (Marglin, H. and Merrifield, R. B., Ann. Rev. Biochem. 39:841-866 (1970). Sequence analysis performed by automated Edman degradation confirmed that the peptide had been correctly synthesized. The final proof that this is the correct epitope has been obtained. Rabbits were immunized with the dodecapeptide coupled to a carrier (bovine gamma globulin in complete Freunds adjuvant). Two weeks after the injection, the rabbits were bled and their serum assayed for the presence of antibodies against the dodecapeptide and against extracts of sporozoites. The results showed that the animals produced high titers (greater than 1:1000) of antibodies to the native CS protein present in the parasite extracts.

Once a clone expressing an immunochemically reactive protein has been identified, the inserted cDNA sequence can be employed as a hybridization probe to identify cDNA coding for sporozoite antigen proteins from other Plasmodium species. The cDNA clone can also be used to screen Plasmodium genomic DNA obtained, for example, from merozoites, to detect DNA sequences coding for sporozoite antigen protein. Therefore, once the first cDNA sequence coding for a sporozoite antigen or fragment thereof is cloned, the subsequent isolation and purification of other species cDNAs is substantially simplified.

### EXAMPLE 2

### Competition between monoclonals for specific antigen

Monoclonal antibodies which bind to distinct areas of an antigen molecule do not interfere with each other; on the other hand, monoclonal antibodies directed against the same or topographically related epitopes or antigenic determinants, will inhibit each other's activity. Thus, it is possible to map the epitopes of an antigen.

The number of epitopes of CS proteins which react with monoclonal antibodies was determined by an immunoradiometric assay performed as follows:

### A) Preparation of plates coated with sporozoite extracts.

Sporozoites were purified from salivary glands of infected mosquitoes as described by Yoshida, N. et al, Science 207, 71 (1980). They were suspended in phosphate-buffered saline (PBS) at a concentration of 10⁶/ml and subjected to sonication (100 W for 3 minutes), then further diluted 20-fold in PBS. Then 50 µl of the suspension were delivered to the bottom of wells of Falcon "3911" microtiter plates, manufactured by Falcon Plastics, Oxnard, California. These were incubated overnight at 4°C and washed with PBS. The wells were then carefully washed with Tween-20 Trademark, Atlas Europol SpA, Ternate, Italy, (0.05% v/v) and incubated for 3 hours in PBS-containing 0.5% (w/v) bovine serum albumin (BSA) to saturate the hydrophobic sites of the plastic.

### B) Preparation of monoclonal antibodies.

Monoclonal antibodies were raised against different species of sporozoites as described by Yoshida, et al, supra; and Potocnjak, P., et al, J. Exp. Med. 151, 1504 (1980). The antibodies were isolated from ascitic fluid of mice injected with the hybridomas by standard chromatographic procedures (ion exchange chromatography and filtration in Sephadex G-200). The purity of the antibodies was ascertained by SDS-PAGE. The antibodies were then radiolabeled with ¹²⁵I using Iodogen (Pierce Chemical Co., Rockford, Ill.) according to the instructions of the manufacturer. The specific activity varied between 10⁷ - 3 X 10⁷ cpm per µg protein.

### C) Titration of monoclonal antibodies.

The minimal concentration of a monoclonal antibody which saturates the antigen sites in the bottom of wells of microtiter plates was determined as follows:

To a series of tubes containing a constant amount (0.5 ng) of radiolabeled monoclonal antibody diluted in PBS-BSA, increasing amounts of cold antibody were added, maintaining a constant total volume. 30 µl aliquots of the various mixtures containing the same number of counts, but different concentrations of antibody, were delivered to the bottom of individual wells of the microtiter plates pre-coated with specific antigen. After incubation for 1 hour at room temperature, the wells were washed with PBS-BSA and counted in a gamma counter. The greatest concentration of monoclonal antibody yielding a maximum of counts bound, represents the saturating dose of monoclonal antibody.

### D) Competition between monoclonal antibodies for binding to the antigen

Several monoclonal antibodies were prepared against the CS proteins of P.knowlesi. The monoclonal antibodies were labeled with ¹²⁵I and the saturating dose determined as described, supra. Then, cross-titrations were performed as follows to determine whether each cold monoclonal antibody interfered with the binding of any other labeled monoclonal antibody to the solid-phase antigen.

To a series of antigen-containing wells, 50 µl of different concentrations of the various cold monoclonals diluted in PBS-BSA were added. The plates were incubated for 1 hour at room temperature. Then, 50 µl of one of the radiolabeled monoclonals (for example, 2G3) at twice the saturating concentration, were added to all the wells. After an additional hour of incubation, the wells were washed and counted. The number of specific counts bound to antigen was calculated as the number of counts bound in wells incubated with 2G3 alone at the saturating dose minus the number of counts bound in wells incubated with 2G3 in the presence of cold 2G3 at a concentration 10³ times the saturating dose. The counts which could not be inhibited by the homologous cold antibody represent non-specific binding. From these numbers, the percentages of inhibition of binding of 2G3 by the other monoclonal antibodies were calculated. The titration was repeated for each labeled monoclonal antibody. The results are summarized in Table 1. It can be seen that all monoclonal antibodies to P.knowlesi strongly inhibit each other, indicating that they must bind to closely related or identical epitopes.

An identical procedure was followed to study the specificities of the monoclonal antibodies to P.vivax and P.falciparum (Tables II and III), P. malariae and P.berghei (not shown). The overall results demonstrate that there is a single immunodominant region in every CS protein.

### EXAMPLE 3

### Competition between monoclonal antibodies and polyclonal antisera for sporozoite antigens.

### A) Assay for the binding of polyclonal antibodies to sporozoite extracts.

The first step of this assay was essentially the same as that described in Example 2; that is, wells of microtiter plates were coated with crude sonicated extracts of sporozoites, washed with Tween-20 and saturated with PBS-BSA. Then, serial dilutions in PBS-BSA of the polyclonal antibodies to the homologous sporozoite species were prepared and aliquots of 30 µl delivered to the bottom of individual microtiter wells. Controls consisted of wells incubated with dilutions of polyclonal antibodies to an unrelated antigen. After an incubation of 4 hours at room temperature, the wells were washed. The presence of antibodies in the wells was detected with a second antibody (¹²⁵I-labeled and affinity-purified) to the immunoglobulin of the appropriate species. For example, in the case of human polyclonal antibodies, the second antibody consisted of 50 µl ( µg/ml) of an affinity-purified rabbit anti-human Ig. The rabbit antibodies were polyspecific, reacting with human kappa, gamma and mu chains, and had been preabsorbed with mouse Ig. This absorption was necessary to prevent the interaction of the developing reagent with the mouse monoclonal antibodies used in the inhibition assay described below. When monkey polyclonal antibodies were used, the developing reagent was similarly prepared from a rabbit antiserum to monkey Ig.

### B) Inhibition of binding of polyclonal antibodies by monoclonal antibodies

The wells coated with sporozoite extract were first incubated with a purified monoclonal antibody to the repetitive epitope of a CS protein, at saturating levels (see Example 2 for the determination of the saturating dose). After incubation for 1 hour at room temperature, the dilutions of the polyclonal antibodies were added, and the assay proceeded as described above.

Inhibition assays have been performed in the following systems:
1) P.knowlesi sporozoite extracts reacting with monkey antisera to X-irradiated P.knowlesi sporozoites. Inhibitory monoclonal antibody 2G3 (Cochrane, A. H., et al, Proc. Nat. Acad. Sci. USA 79, 565 (1982)).
2) P.vivax sporozoite extracts reacting with serum of humans vaccinated by the bite of X-irradiated P.vivax-infected mosquitoes. Inhibitory monoclonal antibody 2F2 (Nardin, E.H. et al, J. Exp. Med. 156:20 (1982)).
3) P.falciparum sporozoite extracts reacting with serum of humans vaccinated by the bite of X-irradiated P.falciparum-infected mosquitoes. Inhibitory monoclonal antibody 2A10 (Nardin, E.H. et al, supra).

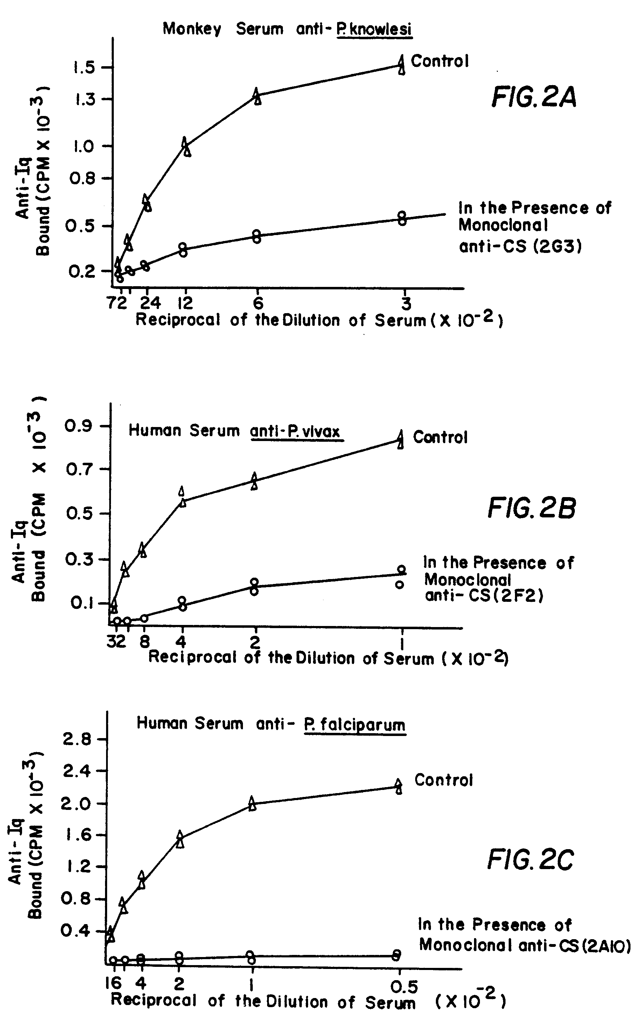

Typical results of these assays are illustrated in Figure 2. In every case, the monoclonal antibodies inhibited 70% or more of the interaction between the extracts and the polyclonal antibodies.

Considering that the solid-phase antigen is prepared by sonication of whole sporozoites and probably contains intracellular as well as plasma membrane proteins, these results indicate that a large proportion of the immune response in the polyclonal sera was directed against the repetitive epitope of the immunodominant region of the CS protein (see infra).

### EXAMPLE 4

### Presence of repetitive epitopes in the immunodominant region of CS proteins from several species of sporozoites.

The experiments in Examples 2 and 3 showed that the CS proteins of four species of Plasmodium contain a single immunodominant region. Shown in this section is the evidence that 1) all immunodominant regions contain a repetitive epitope, and 2) all monoclonals react with a repetitive epitope present in the immunodominant region.

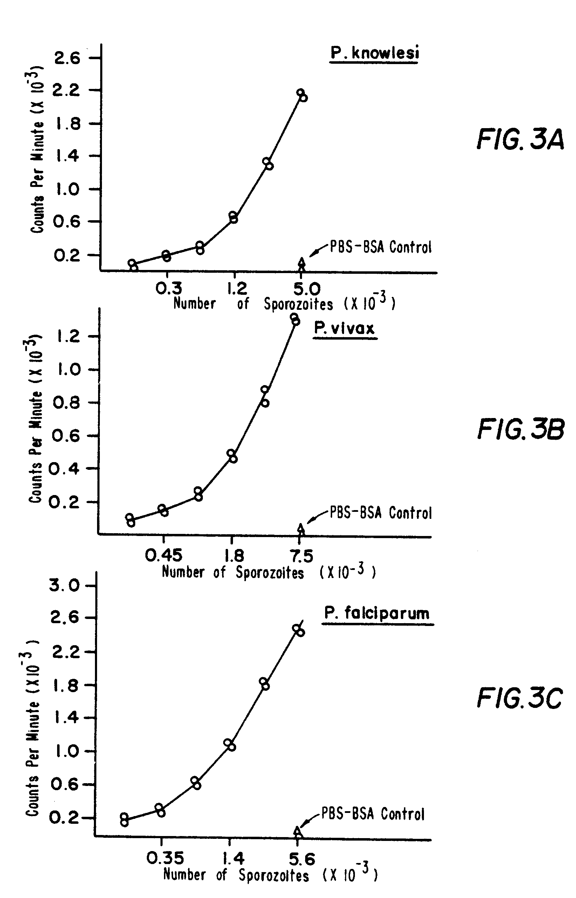

The presence of repetitive epitopes in CS proteins is based on the observation that two-site immunoradiometric assays to measure CS proteins can be performed with a single monoclonal antibody. This is illustrated in Figure 3, for the CS proteins of P.vivax, P.falciparum, and P.knowlesi. Identical results were obtained with the CS protein of P.berghei and the monoclonal 3D11 (not shown) and the CS protein of P.malariae. The assays were performed as follows:
Wells of microtiter plates (Falcon 3911) were incubated overnight at 4°C with 50 µl of a 10 µg/ml solution in PBS of a monoclonal antibody. The wells were washed with PBS and incubated for 2 hours at room temperature with PBS-Tween 20 (0.05% v/v) and for 3 hours at room temperature with PBS-Tween 20-BSA (1% w/v). 30 ul of serial dilutions of extracts of sporozoites were delivered to the bottom of the wells, and the plates incubated overnight in the refrigerator. The extracts had been prepared by treating purified salivary gland sporozoites (10⁷/ml) with 2% (v/v)NP-40 (Trademark, Particle Data Laboratories, Elmhurst, Ill.) in PBS for 2 hours at room temperature, followed by centrifugation at 100,000 g for 1 hour. The dilutions of the extract were made in PBS-BSA containing 0.1% (v/v) NP-40. After incubation, the wells were washed with PBS-Tween 20-BSA. Then 50 µl (about 5-10 ng) of the same ¹²⁵I-labeled monoclonal antibody, diluted in PBS-BSA-Tween 20, were added, and incubation at room temperature proceeded for an additional hour. The wells were washed with PBS-Tween 20-BSA and counted. Controls consisted of wells initially coated with BSA alone. As shown in Figure 3, specific binding was observed in every instance using the homologous antigen. These experiments demonstrate that the various extracts of sporozoites contain CS proteins which are at least divalent, since they can bind two molecules of a single monoclonal antibody, one of them in solidphase, attached to the plastic, and the other in fluid phase and radiolabeled.

It could be argued, however, that the extracts contained aggregated CS protein. This possibility was excluded by the experiments described below, which show directly that the molecular weight of the divalent or multivalent antigen in the extracts corresponded to that of monomers of the CS proteins.

Extracts were prepared as described above treated with SDS 2% (w/v) - 6M urea, and subjected to ultracentrifugation onto 5% (w/v) -20% (w/v) sucrose gradients. The runs were performed in an ultracentrifuge, using a Beckman SW-20 50.1 (Trademark, Beckman Instrument Co., Fullerton, California) rotor at 48,000 rpm for 20 hours. After centrifugation, the bottoms of the tubes were perforated and drops collected in separate tubes. The fractions were analyzed by two methods for the presence of CS protein.
1) Analysis of the fractions by the two-site immunoradiometric assay. This was performed with several monoclonal antibodies for each extract, as described previously in this section. The results of the assays are expressed as number of sporozoite equivalents present in each fraction, as calculated from a standard curve obtained on the same day of the experiment.
2) Analysis of the fractions by inhibition of binding of monoclonals to antigen-coated plates. The gradient fractions were also analyzed by an assay which detects single epitopes on the CS protein. This assay was performed as follows: Antigen-coated plates were prepared, and the minimal saturating dose of radio-labeled monoclonal antibody was determined as described in Example 2. Aliquots of gradient fractions were mixed with the radiolabeled antibody for one hour at room rempterature, and then 30 µl of the mixtures were delivered to the wells containing solid-phase antigen. after an additional hour of incubation, the wells were washed and counted. The inhibition of binding is also expressed as number of sporozoite equivalents present in the fraction, as calculated from a standard curve.

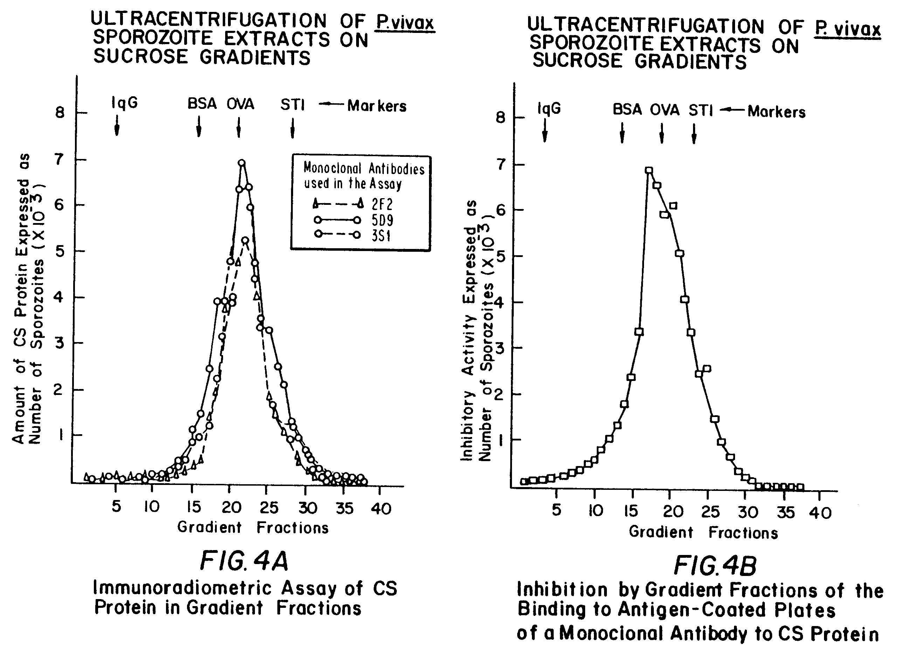

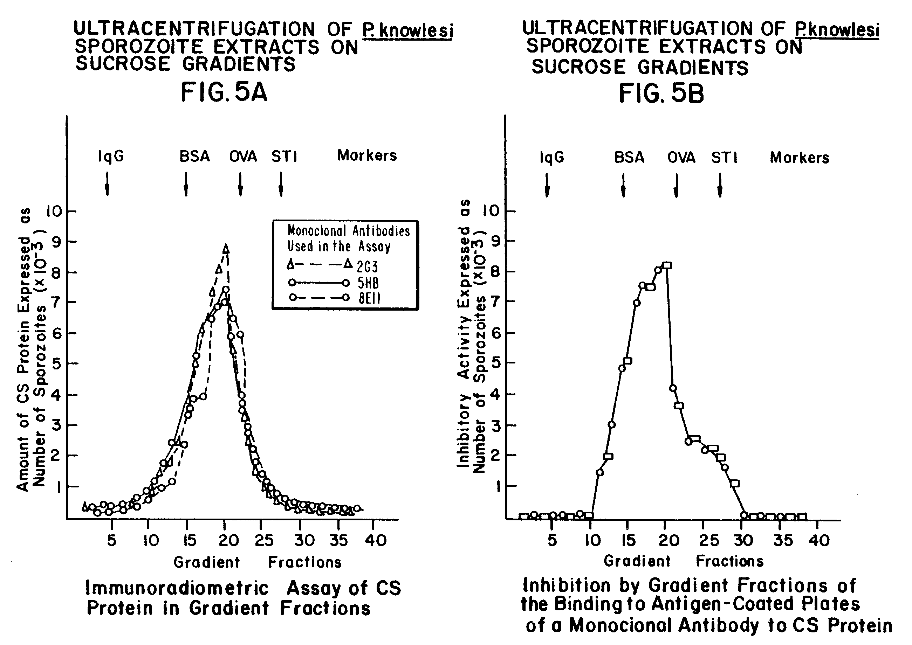

The results of these experiments are summarized in Figs. 4 and 5, which also show the position in the fractions of marker proteins. The results show that the CS antigen of P.vivax and P.knowlesi was detected by both assays and sedimented in a single peak between the markers ovalbumin and bovine serum albumin. The amount of CS protein found in this peak represented 95% or more of the original input. Since CS proteins and their precursors have molecular weights between 40,000 and 60,000, these results strongly suggest that the extracts contain mainly or exclusively, monomers of these molecules. Moreover, all monoclonal antibodies tested recognized a repetitive epitope on the CS protein. The simplest explanation for this finding is that all of them react with the same epitope.

In short, the present results and those of the previous examples, demonstrate that the CS proteins of the human malaria parasites P.vivax, P.falciparum and P.malariae contain a single immunodominant region and repetitive epitopes, as in the case of P.knowlesi.

### EXAMPLE 5

### Cross-reactivity between species of sporozoites

The cross-reactions between the monoclonal antibodies to the repetitive epitopes of the CS proteins from various species of Plasmodium were detected by circumsporozoite (CSP) reactions or by the indirect immunofluorescence test. These tests are described in Nardin, E. H., et al, Nature 274, 55 (1978) and Danforth, H. D., et al, J. Parasitol, 64, 1123 (1978), and were performed with monoclonal antibodies.

For example, the CSP reaction is performed by incubation of dilutions of serum in PBS-BSA with purified viable salivary gland sporozoites at room temperature. After 10 minutes or more of incubation, the sporozoites are examined by phase-contrast microscopy. A positive reaction consists of a thread-like precipitate formed at the posterior end of the parasite. The CSP reaction does not occur in the cold, or with formaldehyde-fixed parasites. As demonstrated by Potocnjak, P., et al, supra, the CSP reaction is caused by the cross-linking of the CS protein by antibodies.

The indirect immunofluorescence test is performed with glutaraldehyde-fixed sporozoites. The parasites are treated with 1% (v/v) glutaraldehyde solution in PBS for 30 minutes at 0°C. Then they are washed in PBS, and incubated overnight with 0.1% (w/v) glycine in water. After washing by centrifugation, the resuspended sporozoites are deposited within 10 µl droplets on microscopic slides, at a concentration of 2 X 10⁶/ml. The droplets are air-dried and kept at -70°C. The assay is performed by incubation of the sporozoites with dilutions of the immune serum for 2 hours at room temperature, followed by washings with PBS, and a new incubation for 2 hours with a second antibody, fluorescein-labeled, directed against the Ig of the first immune serum. The second antibody (for example, rabbitantihuman Ig) can be obtained from a commercial source (Cappel Laboratories, Cochranville, Pa.). After washing, the slides are viewed with a fluorescence-microscope.

Using both procedures, the following cross-reactions were observed between monoclonal antibodies to the repetitive epitopes of the CS protein:
A) anti-P.knowlesi reacted with P.falciparum and P.cynomolgi;
B) anti-P.cynomolgi reacted with P.vivax;
C) anti-P.yoeli nigeriensis reacted with P.berghei. In this case the cross-reactive monoclonal antibodies even neutralized the infectivity of the heterologous species; and
D) anti-P.malariae reacted with P.brasilianum.

### EXAMPLE 6

### Reactivity of synthetic peptides with monoclonal antibodies to the repetitive epitope of P.knowlesi.

The two synthetic peptides described in Example 1 were used in these studies. One of them was composed of the 12 amino acid sequence. H₂N-GlnAlaGlnGlyAspGlyAla-AsnAlaGlyGlnPro-COOH (12-MER) and the other was a dimer of the same sequence (24-MER). The 24-MER (but not the 12-MER) was directly shown by immunoradiometric assay to contain two epitopes of the CS protein of P.knowlesi. The assay was performed as follows:

Wells of microtiter plates (Falcon 3911) were incubated overnight at 4°C with 50 µl of a 10 µg/ml solution in PBS of the monoclonal 2G3 anti-CS protein of P.knowlesi. The wells were washed with PBS and then incubated for 2 hours at room temperature, with PBS-Tween 20 (0.05% (w/v), and then for 3 additional hours with PBS-BSA (1% (w/v). The synthetic peptides were diluted serially in PBS-BSA-Tween 20, and 30 µl aliquots of the dilutions were delivered to the bottom of individual wells. The plates were incubated overnight in the refrigerator, washed with PBS-BSA-Tween 20, and incubated with 5.0 µl (10 ng) of ¹²⁵I-labeled monoclonal antibody 2G3 diluted in PBS-BSA-Tween 20 (about 100,000 cpm). Controls consisted of plates coated with a non-relevant monoclonal antibody of the same isotype as 2G3. The results are shown in Table IV. Specific counts were found only in wells incubated with the 24-MER and they were proportional to the dose of peptide added to the well.

No specific counts of the 24-MER were bound to the wells in which the solid-phase antibody was the monoclonal 3D11, which is directed against the CS protein of P.berghei.

These results strongly suggest that the 24-MER contains two identical epitopes recognized by the monoclonal 2G3, and that the 12-MER contains either one epitope or none. To distinguish between these possibilities, an assay was conducted to determine the ability of the 12-MER to inhibit the interaction between the CS proteins of P.knowlesi and the monoclonal antibody 2G3. The inhibition assay was performed as follows:
Wells of microtiter plates were coated with the monoclonal antibody 2G3 washed with Tween-20, saturated with BSA as described supra. In one series of tubes, the 12-MER was serially diluted in PBS-BSA-Tween 20. In a second series of tubes, an extract of P.knowlesi sporozoites (prepared as described by Cochrane, et al, supra) was serially diluted in PBS-BSA-Tween 20. Aliquots of each dilution of sporozoites were mixed with equal volumes of all dilutions of 12-MER. 30 µl of these mixtures were then added to the bottom of the 2G3-coated wells. As positive and negative controls, 30 µl aliquots of sporozoite dilutions mixed with PBS-BSA-Tween 20 were added to other wells which had been precoated with 2G3 or with a non-relevant monoclonal antibody. After overnight incubation in the refrigerator, the wells were washed and 50 µl (10 ug) of ¹²⁵I-labeled 2G3 diluted in PBS-BSA-Tween 20 were added. Following an additional incubation at room temperature for 1 hour, the wells were washed and counted in a gamma counter. The results are shown in Table V. The 12-MER inhibited, in a dose-dependent fashion, the interaction of the P.knowlesi CS protein with 2G3.

The conclusion drawn from this experiment is that the 12-MER peptide contains an epitope of the CS protein of P.knowlesi.

The reactivity of the synthetic peptides was confirmed by radiolabeling the 24-MER and showing that it bound specifically a monoclonal antibody to P.knowlesi (5H8). This experiment was performed as follows:
A) Preparation of Sepharose-4B (Trademark, Pharmacia, Inc. Uppsala, Sweden) coupled to the monoclonal antibodies 5H8 and 3D11, directed against the CS proteins of P.knowlesi and P.berghei respectively.
   The antibodies were coupled to CNBr Sepharose beads (Pharmacia Fine Chemicals Uppsala, Sweden) following the instructions of the manufacturer. After coupling, the beads (containing about 10 mg antibody/ml) were treated for 1 hour at room temperature with 0.5% glutaraldehyde (to prevent leakage of the proteins), then with a solution of 10 mg/ml glycine in PBS, and finally resuspended at 20% volume in PBS-BSA (1% (w/v)) Tween 20 (0.05% (v/v)). The Sepharose-coupled monoclonals were designated Sepharose-5H8 and Sepharose 3D11, respectively.
B) Radiolabeling of the 24-MER was performed with ¹²⁵I using the Bolton-Hunter reagent (Amersham International Ltd., Amersham, U.K.) according to the instructions of the manufacturer, using 10 µl of a solution of the 24-MER (10 mg/ml) and 0.1 millicuries of the Bolton-Hunter reagent. The peptide was purified after labeling in a Sephadex-G10 column equilibrated in PBS-gelatin (0.2% (w/v)). Presuming that 100% of the peptide was recovered, the specific activity was 10⁵-cpm/µg of protein.
C) Specific binding of the radiolabeled 24-MER to Sepharose-5H8.

Four 200 µl aliquots of the 20% (w/v) suspension of Sepharose-5H8 were added to tubes containing 150 µl of a dilution of the labeled 24-MER in PBS-BSA-Tween 20 (45,000 cpm). To two of the tubes 50 µl of diluent were added. To the other two tubes 50 µl of cold 24-MER (500 µg) diluted in PBS-BSA-Tween 20 were added. The tubes were incubated overnight in the refrigerator. The beads were washed by centrifugation and counted.

As controls, identical mixtures were prepared in tubes containing Sepharose-3D11. The results are shown in Table VI. These results demonstrate that the radiolabeled peptide bound specifically to the monoclonal antibody 5H8 anti-CS protein of P.knowlesi. In addition, it appears that the 24-MER may have interacted weakly with the 3D11 antibody. This is not surprising, considering the evidence that all CS proteins are structurally related, and that, in these experiments, the molar ratio of antibody to the ligand peptide was quite high.

### EXAMPLE 7

### Immunization with the synthetic repeated epitope of P.knowlesi (24-MER)

The synthetic 24-MER is synthesized as described in Example 1, except that a cysteine residue is added at the N-terminus. To determine whether the synthesis has been performed correctly, an aliquot is subjected to acid hydrolysis at reduced pressure (6M HCl, 110°C, 72 hours) and its amino acid composition is determined. The peptide is coupled to a carrier protein either keyhole limpet hemocyanin, or tetanus toxoid, through its N terminal cysteine residue, using m-maleimidolbenzoyl-N-hydroxy-succinimide ester (MBS) as the coupling reagent (Ling, F. T., et al, Biochemistry 18, 690 (1979)). This is a bifunctional reagent which under appropriate conditions reacts specifically with the amino group of the carrier on the one hand, and with the thiol group of the peptides, on the other hand.

4 mg of the carrier protein in 0.25 ml of 0.05M PO₄ buffer, pH 7.2, is reacted dropwise with 0.7 mg MBS dissolved in dimethyl-formamide, and stirred for 30 min. at room temperature. The product, that is, MB-carrier, is separated from the unreacted chemicals by passage in a Sephadex G-25 column equilibrated in 0.05M PO₄ buffer, pH 6.0. The MB-carrier is then reacted with 5 mg of the 24-MER containing cysteine, dissolved in PBS (pH 7.4). The mixture is stirred for 3 hours at room temperature. Coupling efficiency is monitored with radioactive peptide; that is, a trace amount of ¹²⁵I-labeled 24-MER is mixed with cold peptide during the synthesis. Dialysis of the conjugate permits evaluation of the proportion of incorporated label. The number of 24-MER groups per 100,000 M.W. carrier was estimated to be about 10-14.

Five rhesus monkeys are immunized with 200 mg of the conjugated protein adsorbed to aluminum hydroxide gel. Their serum is monitored for the presence of antibodies to CS proteins of P.knowlesi by the immunoradiometric assay described in Example 3. That is, serum dilutions are incubated with antigen-coated wells of microtiter plates. The presence of monkey antibody bound to the solid-phase antigen is monitored by incubation with ¹²⁵I-labeled affinity-purified rabbit-anti-human Ig (which strongly cross-reacts with rhesus monkey Ig).

After 30 days, the serum titers of the monkeys rise to titers of greater than 1/1000. At this time, these monkeys (as well as five other control monkeys injected with non-conjugated carrier protein adsorbed to aluminum hydroxide) are challenged with 2,000 viable P.knowlesi sporozoites. The infection is monitored daily for a total of 30 days by microscopic examination of blood smears, starting one week after the inoculation of the parasites. The results show that the five monkeys immunized with the vaccine (conjugated protein) are totally protected; that is, no parasites are found in their blood. In contrast, the control monkeys have trophozoites of P.knowlesi in the circulation 7-12 days after challenge.

### EXAMPLE 8

### Immunization with the synthetic repeated epitopes of P.vivax and P.falciparum

The synthetic sequences corresponding to the repeated epitopes of P.vivax and P.falciparum are synthesized essentially as described in Example 1, except that cysteine residues are added at the N-terminals as described in Example 7. To determine whether the synthesis has been performed correctly, aliquots are subjected to acid hydrolysis at reduced pressure (6M HCl, 110°C, 72 hours) and their amino acid composition is determined. The peptides are coupled to a carrier protein either keyhole limpet hemocyanin, or tetanus toxoid, through its N terminal cysteine residue, by using MBS as the coupling reagent, as described in Example 7.

4 mg of the carrier protein in 0.25 ml of 0.05M PO₄ buffer, pH 7.2., is reacted dropwise with 0.7 mg MBS dissolved in dimethyl-formamide, and stirred for 30 min. at room temperature. The product, that is, MB-carrier, is separated from the unreacted chemicals by passage in a Sephadex G-25 column equilibrated in 0.05M PO₄ buffer, pH 6.0. The MB-carrier is then reacted with 5 mg of each peptide containing cysteine, dissolved in PBS (pH 7.4). The mixture is stirred for 3 hours at room temperature. Coupling efficiency is monitored with radioactive peptide; that is, a trace amount of ¹²⁵I-labelled is mixed with cold peptide during the synthesis. Dialysis of the conjugate permits evaluation of the proportion of incorporated label. The number of synthetic peptides per 100,000 M.W. carrier is estimated to be about 10-14.

Five chimpanzees are immunized with 200 µg of each of the conjugated proteins adsorbed to aluminum hydroxide gel. Their serum is monitored for the presence of antibodies to CS proteins of P.vivax and P.falciparum by the immunoradiometric assay described in Example 3. That is, serum dilutions are incubated with antigencoated wells of microtiter plates. The presence of chimpanzee antibody bound to the solid-phase antigen is monitored by incubation with ¹²⁵I-labeled affinity-purified rabbit-anti-human Ig (which strongly cross-reacts with chimpanzee Ig).

After 30 days, the serum titers of the chimpanzees rise to titers of greater than 1/1000. At this time, these chimpanzees (as well as five other control chimpanzees injected with non-conjugated carrier protein adsorbed to aluminum hydroxide) are challenged with 2,000 viable P.vivax sporozoites. The infection is monitored daily for a total of 30 days by microscopic examination of blood smears, starting one week after the inoculation of the parasites. The results show that the five chimpanzees immunized with the vaccine (conjugated protein) are totally protected; that is, no parasites are found in their blood. In contrast, the control chimpanzees have trophozoites of P.vivax in the circulation 10-12 days after challenge.

Next, a second challenge with 10,000 P.falciparum sporozoites is given to the same chimpanzees. Again the vaccinated apes are protected, while the controls are all infected.

Based upon the close similarities of human and chimpanzee immune responses and on the fact that protective immunity has been obtained in humans by injection of inactivated sporozoites of P.falciparum and for P.vivax, the results obtained upon immunization of chimpanzees with the described synthetic peptide will also be obtained following similar treatment of human patients.

A more precise identification of the immunodominant region in fact of the immunodominant epitope itself, is desirable for several reasons: one is that such identification would be useful in providing a further understanding of the immunogenicity of the CS protein; another is that a peptide with a shorter amino acid sequence identical or related to that of the epitope is easier to prepare and/or purify, using either conventional peptide synthesis or recombinant DNA techniques. Such a peptide would be useful if it displayed anti-CS binding activity similar to that of the dodecapeptide. Of even greater interest, was the verification of whether the epitope was represented within an uninterrupted sequence of amino acids in the dodecapeptide or whether it was configurational, i.e., formed by residues juxtaposed by virtue of the higher order structure of the dodecapeptide (12-peptide). Since several monoclonal (and polyclonal) antibodies to the CS protein neutralize the infectivity of sporozoites, if the epitope was represented in an uninterrupted sequence and was formed of a relatively short sequence, this peptide would provide the basis for the development of a synthetic vaccine.

The size and shape of epitopes found in carbohydrate antigens have been extensively studied, but less is known about the structure of epitopes from protein molecules. Some epitopes of protein antigens have been defined at the level of their 3D structure. In every instance, the epitopes were formed not by the primary sequences alone, but by the juxtaposition of residues brought together by the folding of the polypeptide chain(s) of the native molecule. For example, monoclonal antibodies to sperm whale myoglobin did not react with any of the three CNBr cleavage fragments which collectively encompass the whole sequence of hemoglobin (Berzofsky, J.A. et al., "Topographic Antigenic Determinants Recognized by Monoclonal Antibodies to Sperm Whale Myoglobin" (1982) J. Biol. Chem. 257: 3189-3198 ; East, I.J. et al, "Antigenic Specificity of Monoclonal Antibodies to Human Myoglobin" (1982) J. Biol. Chem. 257: 3199). A monoclonal antibody bound to the epitope in the lysozyme was found to include a region containing the Arg 68 - Arg 45 complex which borders the catalytic site (Smith-Gill, S.J. et al., "Mapping the Antigenic Epitope for a Monoclonal Antibody Against Lysozyme", (1982) J. Immunol. 128: 314). Monoclonal antibodies which recognize the A-chain loop (A 8-10) of insulin failed to bind to isolated A chains, or to synthetic peptides (Shroer J.A. et al., (1983) Eur. J. Immunol. 13: 693).

The approach used herein involved starting with the amino acid sequence of the repetitive peptide of a sporozoite CS protein such as that of Plasmodium knowlesi, synthesizing a tandem repeat peptide thereof (twice the number of aminoacids) and analog peptides thereof having progressively smaller sequences (by progressive omission of terminal amino acids), and determining the reactivity of monoclonal antibodies to the CS protein (monoclonal anti-CS) for such peptides and each of their analogs. The objective was to find the shortest analog with high antibody reactivity, thus simultaneously identifying the location of the epitope within the dodecapeptide (if the locus of such epitope was in the primary amino acid sequence) and identifying a peptide having immunochemical reactivity vis-a-vis monoclonal anti-CS similar to that of the dodecapeptide.

The particular tandem repetitive polypeptide chosen for the experimental work described below was that of P. knowlesi CS protein. This repetitive polypeptide is a dode-capeptide having the amino acid sequence (from N to C terminus) (Gln₁-Ala₂-Gln₃-Gly₄-Asp₅-Gly₆-Ala₇-Asn₈-Ala₉-Gly₁₀-Gln₁₁-Pro₁₂).

Several monoclonal anti-CS have been raised against the P. knowlesi CS protein (as described in Patent Application No. 234,096) and were available for testing the immunoreactivity of peptides synthesized herein. However, the fact that both the peptides synthesized or employed in the present work and the antibodies relate to P. knowlesi, does not limit the applicability of the present invention to P. knowlesi CS protein. Strong evidence exists that CS-proteins of other Plasmodium species also contain tandem repeating peptides and constitute proteins related to the P. knowlesi CS protein (even though their amino acid sequences may not be the same). For example, previously reported results of immunological assays indicated that only one area of the CS molecule of P. vivax, P. falciparum, P. knowlesi and P. berghei was recognized by all monoclonal anti-CS (raised from CS protein of the same species). In addition, cross-reactivity has been reported between CS proteins of different Plasmodium species and P. knowlesi anti-CS (Cochrane et al (1982) Proc. Nat'l Acad. Sci. USA 79:5651). Moreover, Santoro et al (1982) J. Biol. Chem. 258:3341, have reported evidence that the CS proteins of different malaria species are part of a family of structurally related molecules. Thus, it is anticipated that the present invention will be applicable to several Plasmodium species.

A synthetic dodecapeptide (corresponding to the P. knowlesi CS protein repeating peptide sequence) and a tetraeicosapeptide consisting of a tandem repeat of said dodecapeptide were prepared and tested for anti-CS binding activity with six anti-CS which had been raised following immunization of mice with intact sporozoites, but which were shown to be reactive with a single molecule, the CS protein: Cochrane, A.H. et al, "Monoclonal Antibodies Identify the Protective Antigens of Sporozoites of Plasmodium knowlesi" (1982) Proc. Nat'l. Acad. Sci., USA, 79, 5651. The CS protein is stage-and species-specific (Vanderberg, et al, Military Medicine, 304:1183 (1969); Cochrane, A.H., et al, "Antibody Induced Ultrastructural Changes of Malarial Sporozoites" (1969), J. Immunol. 116, 859) distributed uniformly over the entire sporozoite surface and is shed when cross-linked by antibodies Potocnjak, P. et al, "Monovalent Fragments (Fab) of Monoclonal Antibodies to a Sporozoite Surface Antigen" (1980) J. Exp. Med., 151, 1505.

Only one area of the CS molecule of several species was recognized by all homologous monoclonal anti-CS and that this immunodominant region was multivalent with regard to the expression of a single epitope: Zavala, F., et al., (1983) J. Exp. Med. 157: 1947. DNA analysis has shown that the immunogenic region of P. knowlesi CS protein consists of a tandem repeat by 12 units of 12 amino acids each (see Peptide Antigen Application). Moreover, the epitope recognized by one monoclonal anti-CS (2G3) was included within a single subunit. The synthetic dodecapeptide very effectively inhibited the anti-CS/CS reaction while the 24/MER interacted simultaneously with two molecules of 2G3.

The present work demonstrated that the epitopes reacting with five other monoclonal anti-CS are also represented within the 24/MER. This was useful in determining whether the epitope lay within a sequence of 24 amino acids, prior to testing whether the epitope was contained within the sequence of the 12 amino acids.

Four monoclonal antibodies (2G3, 5H8, 8B8, and 8E11) were used in immunoactivity testing of synthesized shorter length analogs of the 12-peptide. The purpose was further localization and identification of the epitope contained therein.

An 11-amino acid analog was first synthesized, namely analog (2-12) by omission of the N-end Gln residue. The reactivity of (2-12) was compared with that of the (1-12) and found to be substantially equivalent (see Table IX). Subsequently, the reactivities of 3-12, 4-12 and 5-12 were determined and found substantially identical to that of 1-12. However, omission of the 5th amino acid, residue Asp, resulted in a considerable drop in reactivity. It was tentatively concluded that amino acid residues 1 through 3 and possibly 4 did not contribute to the immunoreactivity of the 12-peptide.

Starting from the C-end, the analogs (1-11), (2-11) and (3-11) were not synthesized based on the hypothesis that amino acids 1, 2 and 3 did not contribute to immunoreactivity. (4-11) had immunoreactivity substantially equivalent to (actually, slightly higher than) that of (5-12). However, (5-11) showed a drop in reactivity commensurate with that observed by going from (5-12) to (6-12).

Shorter length peptides showed no reactivity, thus confirming that the (4-11) and (5-12) peptides were those having high reactivity and minimum length.

The above method of omitting one end amino acid at a time and testing the reactivity of the resulting peptide leads to the isolation of the epitope in the minimum number of steps. Once an amino acid residue is shown not to participate in the epitope, it can be eliminated from further testing.

As summarized in Table IX, the dodecapeptide NH₂Gln-Ala-Gln-Gly-Asp-Gly-Ala-Asn-Ala-Gly-Gln-ProCONH₂ (1-12), as well as the peptides (2-12), (3-12), (4-12) and (5-12) were very efficient inhibitors. However, following removal of Asp or Asp and Gly (positions 5 and 6), the degree of reactivity was considerably lower. This is consistent with the hypothesis that peptides shorter than 5-12 are not active. The role of amino acids from the C-terminal end of (1-12) was also examined.

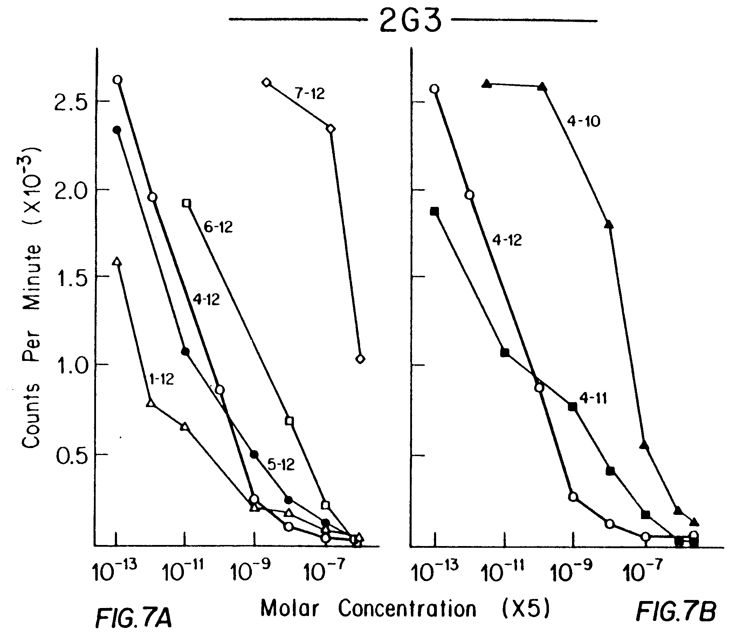

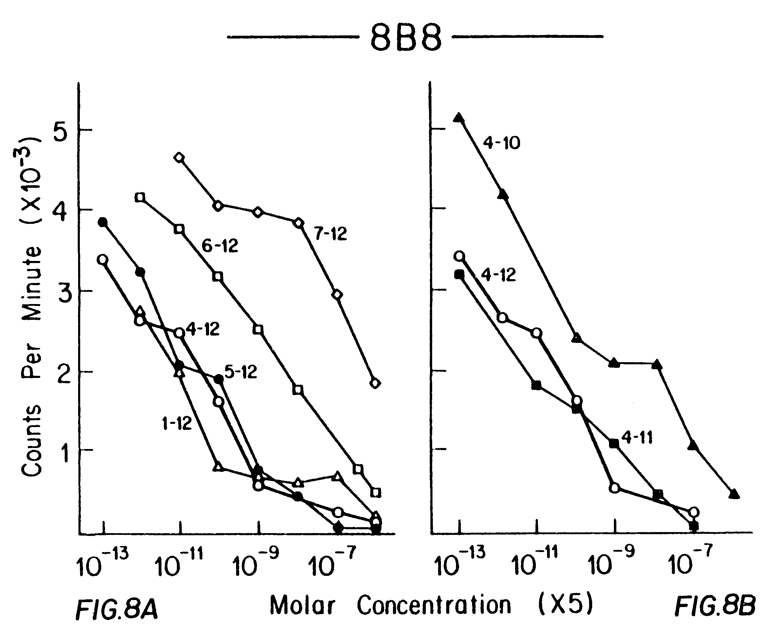

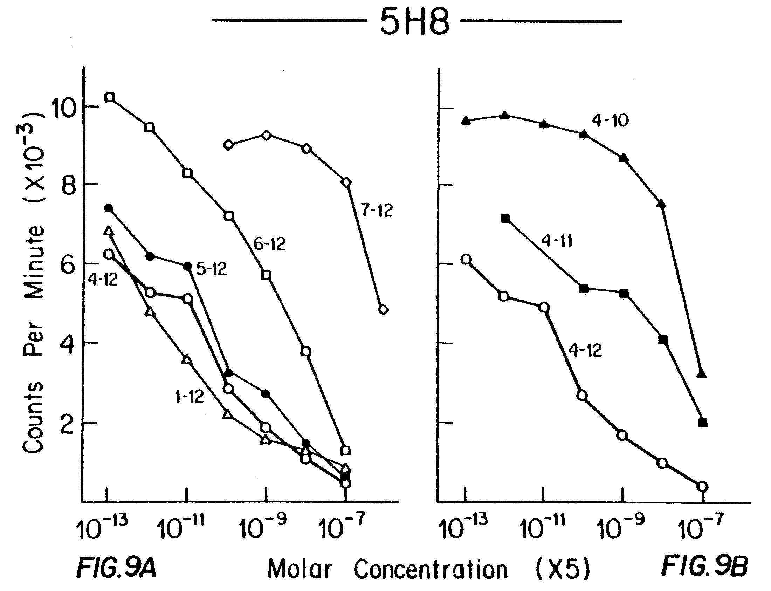

Figure 8 is a graph depicting the inhibition of binding of radiolabelled monoclonal antibody 5H8 to P. knowlesi CS protein in the presence of increasing concentrations of various peptides, said peptides being designated as in Figure 2. As shown in panel B of Figure 8, removal of Pro (position 12) or Gln (position 11) from the dodecapeptide had a considerable effect in its reactivity with the monoclonal 5H8. However, for monoclonals 2G3 and 8B8, the reactivity with 4-12 or 4-11 was almost identical, while 4-10 was much less reactive (panel B, Figures 7 and 9).

Figure 7 is a graph depicting the inhibition of the binding of monoclonal antibody 2G3 to P. knowlesi CS protein by peptides which are analogs of the repetitive peptide of the CS proteins, said peptides being designated by the position of their terminal amino acids within the sequence of the dodecapeptide.

Figure 9 depicts inhibition of binding of radio-labelled monoclonal antibody 8B8 to P.knowlesi CS protein in the presence of increasing concentrations of various peptides, designated by their position within the dodecapetide as in the previous figures.

The dodecapeptide contains only one proline (at the C-terminus) and this residue is not essential for reactivity with two monoclonal antibodies.

The above results led to the conclusion that
(a) contrary to the results of investigation on many other antigens, there is a single immunodominant region in the CS protein of P.knowlesi and the epitope is located within a primary sequence (rather than a conformation) of 12 amino acids around residues 4-11; and
(b) it is possible to obtain substantially equivalent immunoreactivity from a peptide having only an 8-residue rather than a 12-residue sequence; i.e. it is possible to obtain an immunoreactive peptide of minimum amino acid length, shorter than that of the repeating unit (dodecapeptide) itself.

From the above, it is clear that the monoclonals react with a sequence of amino acids around residues (4-11).

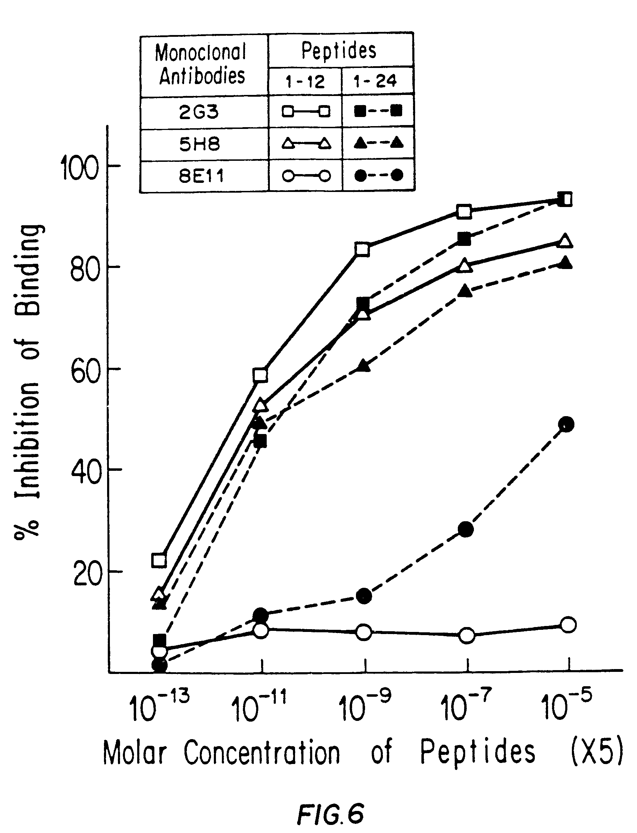

Figure 6 is a graph depicting the relation between the activity of the dodecapeptide (constituting the repeating unit of the repetitive peptide of the P. knowlesi CS protein) and a 24-peptide (consisting of a tandem repeat of said dodecapeptide) inhibiting the binding of different monoclonal antibodies to the P. knowlesi CS protein. Figure 6 shows that the specificity of 8E11 differs markedly from that of the other monoclonal antibodies in that the interaction of 8E11 with the CS protein is only inhibited by (1-24), and not by (1-12). To examine the possibility that 8E11 recognizes a sequence overlapping between two tandem dodecapeptides of 1-12, several other peptides were synthesized and assayed for inhibitory activity, with negative results. This indicates that 8E11 reacts with a configurational or topographic epitope formed by joining two dodecamers.

Three of the four monoclonal antibodies used in the immunoreactivity tests of the 12-peptide, the 24-peptide and the synthetic analogs (2G3, 5H8 & 8E8) were recognized by the reactive shorter analogs of minimum length. Because the epitope is repeated twice within a sequence of 24 consecutive amino acids, it is not likely that the binding sites recognize secondary or tertiary structures of the polypeptide chain. The striking immunogenicity of this epitope is most likely a reflection of the unusual structure of the CS protein, half of which consists of tandem repeats of 12 amino acids, each repeat containing a potential epitope. It is noteworthy that the streptococcal M protein type 24 also contains a repeated peptide subunit, which contains the immunodominant epitope (Beachey, E.H., et al., "Primary Structure of Protective Antigens of Type 24 Streptococcal M Protein", (1980) J. Biol. Chem. 255: 6284-6289. However, the CS-protein is not known to bear any structural or other similarity to the M-protein.

In other studies, monoclonal antibodies 2G3 and 8E11 (or the corresponding Fab fragments) not only bound to the P. knowlesi CS protein, but also neutralized the infectivity of the sporozoites (Cochrane, A.H., et al., supra). In light of the present results, it seemed possible that polyclonal antibodies to synthetic peptides representing the repetitive epitope of the CS protein of P. knowlesi could have similar biological activities. Indeed, rabbits and mice have been successfully immunized against P. knowlesi with the tetraeicosapeptide (1-24) conjugated to a carrier protein. Several animals made antibodies that reacted with the membrane of sporozoites, and immunoprecipitated the CS protein. In addition, sporozoites lost their infectivity when incubated in the serum from one of the immunized rabbits. These observations raised hopes that if equivalent peptides from the CS proteins of human malaria parasites are found to be immunogenic in vivo, they may be used in the formulation of vaccines for humans.

The general steps of the peptide synthesis techniques used herein are well known. Specific modifications made by the present inventors, to adapt such techniques to the synthesis of the present peptides, are described in the Examples.

After synthesis, cleavage and purification, the peptides were tested for reactivity with monoclonal anti-CS, preferably partially purified. The anti-CS used in these tests were produced by ascites tumor induction using hybridoma cells resulting from the fusion of plasmacytoma cells with spleen cells of a mouse immunized with the parasite, as described by Cochrane, A.H. et al, supra, and as disclosed in the Malaria Vaccine Patent Application.

The immunoactivity of the synthetic peptides was evaluated by measuring their ability to inhibit the reaction between the monoclonal anti-CS and the antigen (CS protein). Antigen was purified from sporozoites, as disclosed in the Malaria Vaccine Patent Application and as also described by Vanderberg, supra, and Zavala, F., et al, supra, 1982, is preferably employed using ¹²⁵I-labeled antibody.

This aspect of the present invention is further described below in the following Examples, which are intended to illustrate it but not to limit its scope.

### Materials and Sources

derivatized amino acids (and protective groups) and benzhydrylamine resin: Beckman Instruments, Palo Alto, Calif. hydroxybenzotriazole: Sigma Chemical Co., Inc. St. Louis, Mo. Sephadex G-25 & G-200: Pharmacia Fine Chemicals Co. Piscataway, N.J. bovine serum albumin: Sigma Chemical Co., St. Louis, Mo. Iodogen: Pierce Chemical Co., Rockford, Ill. Boulton-Hunter Reagent: Amersham Corp., Arlington Hgts, Ill.

### EXAMPLE 9

### Peptide Synthesis

Syntheses were carried out using a benzhydrylamine (BHA) resin (0.654 meg/gm) on a Vega model 250C (Vega Biochemicals, Inc., Tuscon, Arizona) automated synthesizer controlled by a Motorola computer with a program based on that of Merrifield, R.B., Fed. Proc. 21:412 (1962); J. Chem. Soc. 85:2149, (1963). First, the dodecapeptide Gln-Ala-Gln-Gly-Asp-Gly-Ala-Asn-Ala-Gly-Gln-Pro was assembled on 3.0 gms of the benzhydrylamine resin which were suspended in CH₂ Cl₂ and washed 3 times with CH₂Cl₂, 3 times with ethanol, and 3 times with CH₂Cl₂ in the synthesizer. The resin was washed for a total of 2 minutes and then treated with 50% trifluoroacetic acid containing 10% anisole in CH₂Cl₂ for 30 min, washed ten times with CH₂Cl₂, neutralized by washing twice with 10% diisopropylethylamine in CH₂Cl₂. The first BOC-amino^{1/} acid was coupled for one hour to the benzhydrylamine resin using three-fold molar excess of BOC amino acid dicyclohexyl carbodiimide in the presence of 3 molar excess of CH₂Cl₂ and hydroxybenzotriazole. Additional aliquots, one of hydroxybenzotriazole and one of diisopropylethylamine, were added at a three-fold molar excess of BOC amino acid for an additional hour. The resin was then washed with CH₂Cl₂ (3 washes), absolute ethanol (3 washes) and CH₂Cl₂ (3 washes) and an aliquot of the mixture was tested using the Kaiser ninhydrin procedure (Kaiser, E. et al., (1970), Analyt. Biochem. 34:595. The resulting peptide was Boc-Gln(NPE)-Ala-Gln(NPE)-Gly-Asp(OBZ)-Gly-Ala-Asn(NPE)-Ala-Gly-Gln(NPE)-Pro-Co-BHA^{2/}. At this point in the synthesis, 50% of the protected peptide resin was removed and saved for HF cleavage and purification.
1/ BOC stands for tertiary butyloxycarbonyl
2/ NPE stands for nitrophenylester OBZ stands for o-benzyl

The tandem repeat; i.e., the tetraeicosapeptide (1-24) was assembled by the sequential addition of protected amino acids in the same order as for the dodecapeptide, using the above-described method.

Following synthesis, 2.0 gms each of the (1-12) and (1-24) protected peptide resins were subjected to treatment with HF as described below and the deprotected cleaved peptides were washed separately with anhydrous ether and extracted with alternate washes of glacial acetic acid and water.

Cleavage of the peptide-resins (2gms each) was performed in a Penninsula HF apparatus (Penninsula Laboratories, San Carlos, Calif.) in the presence of anisole (1.2 ml/mg resin) and methylethyl sulfide (1 ml/mg) at 0°C for one hour after which the mixture was thoroughly dried under high vacuum. The mixture was then washed with cold anhydrous ether, extracted with alternate washes of water and glacial acetic acid and lyophilized.

The crude peptides were then desalted by gel filtration on Sephadex G-25 (120 x 2.0 cm) in 200 mg aliquots. The column was equilibrated with 0.1 MNH₄HCO₃ pH 8.0, also used as the sample buffer. Column effluent was monitored by UV absorbance at 254 and 206 nm with an LKB UV-Cord III monitor.

The peptides thus synthesized, were analyzed and characterized as follows:
Samples were hydrolyzed in 5.7 N HCl for 22 hours at 110°C, dried, reconstituted in 0.2 N sodium citrate, pH 2.2 and applied to the amino acid analyzer (Liquimat III) according to the method of Spackman, D.H. et al, Anal. Chem., 30:1190, 1958.

At selected steps during synthesis, aliquots of the peptide resin were removed from the reaction vessel of the synthesizer mixed with glass beads, subjected to automated solid phase Edman degradation (Laursen, R. (1971) Eur. J. Biochem. 20:89) on a Sequemat Mini 15 peptide sequencer (Seguemat, Inc. Waltham, Mass.). Following cleavage of the peptide from the resin, crude and purified synthetic peptides were subjected to automated liquid phase sequence analysis (Edman P. and Begg G., Eur. J. Biochem. 1:80-90 (1957)) on a Beckman (Model 890) sequencer using a "DMAA" (dimethylallylamine) peptide program to detect the presence of failure sequence and side chain protecting groups on the peptide not removed by HF cleavage. Quantification of the amount of error peptides and side chain protecting groups were assessed by sequence analysis, followed by identification and quantification of the PTH amino acids by high performance liquid chromatography, a well accepted method of "preview" analysis as disclosed by Niall, H.D., et al., "Chemistry & Biology of Peptides: Proceedings of the Third American Peptide Symposium (1972) (J. Meienhofer, ed.) Ann Arbor Science Pub., 695 1972; modified by Tregear, G.W., "Peptides: Proceedings of the Third European Peptide Symposium" (Y. Wolman, ed.,) (1974; and Tregear, G.W., et al., Jr. Biochem. (1977) 16: 2817; as further modified by (Simmons, J. and Schlesinger, D. H., "High-Performance Liquid Chromatography of Side-Chain-Protected Amino Acid Phenylthiohydrantoins", (1980) Anal. Biochem., 104, 254; and Schlesinger, D.H., (1983), Meth. Enzymol. 91, 494.

In addition to the dodecapeptide and 24-peptide, four groups of peptide analogs of the dodecapeptide were synthesized, each possessing as the C-terminal amino acid either Pro, Gln, Gly or Ala, which correspond to positions 12, 11, 10 and 9 respectively, of the dodecamer (Table VII). The peptide analogs possessing Pro at their C-terminus were synthesized by removing dried protected peptide resin (approximately 10% at each step) at positions 10, 8, 7, 6, 5, 4, 3 and 2 during the synthesis of the dodecapeptide, as described above. This procedure yielded 9 protected peptide resins.

Similarly, two peptides were synthesized containing the C-terminal amino acid Gln; i.e., peptides comprising residues in positions 5-11 and 4-11 of the dodecamer. Only one peptide containing Gly at its C-terminus was synthesized, and it corresponded to positions 4-10 of the dodecamer. Finally, four peptides possessing Ala at their C-terminus (positions 7-9, 6-9, 5-9 and 4-9 of the dodecapeptide) were synthesized in analogous fashion. One peptide which bridges parts of two epitopes was synthesized, i.e., Ala₇-Asn₈-Ala₉-Gly₁₀-Gln₁₁-Pro₁₂-Gln₁-Ala₂-Gln₃-Gly₄-Asp₅-Gly₆ to determine if monoclonal antibody which might not recognize the dodecapeptide or any of the above analogs might be directed against a sequence containing parts of two tandem repeats.

These protected peptide-resins were then cleaved and deprotected with HF, desalted on Sephadex G-25 and characterized by amino acid composition and sequencing preview analysis (see Table VII, below). The peptides were used in immunological studies without further purification.

### EXAMPLE 10

### Reaction of Monoclonal Antibodies with the Tetraeicosapeptide

Monoclonal antibodies against surface antigens of sporozoites of the simian malaria parasite Plasmodium knowlesi were produced by fusion of plasmacytoma cells with spleen cells of a mouse immunized with the parasite as previously described by Cochrane et al., supra., (1982) and described in detail in the Malaria Vaccine Patent Application. The monoclonal antibodies (four idiotypes: 2G3, 8B8, 5H8 and 8E11 were partially purified from ascites of mice bearing hybridomas by 50% ammonium sulphate precipitation followed by molecular sieve chromatography on Sephadex G-200.

All types of monoclonal antibodies which were raised against P.knowlesi, and selected for reactivity with the surface membrane of the parasite (Cochrane et al., 1982) reacted with the tetraeicosapeptide.

Competitive inhibition of the antigen-antibody reaction by the thus synthesized peptides was measured by the following radioimmunoassay. P.knowlesi sporozoite CS protein was used as the antigen.
a) Preparation of antigen: 3,000 purified P. knowlesi sporozoites (Vanderberg et al., Military Medicine 134:1183, 1969) in 50 µl of phosphate buffered saline (PBS) containing protease inhibitors, were delivered to the bottom of polystyrene (Falcon 3911 plate, B-D and Co., Oxnard, Calif.) microtiter plates, which were sealed with parafilm and frozen at -70°C for at least ten minutes. The plates were then allowed to defrost slowly at room temperature. This procedure was repeated six times. Then the plates were incubated at 4°C overnight, and at 56°C for five minutes. The P. knowlesi extract was then removed from the wells and these were washed three times with PBS containing 1% bovine serum albumin (BSA) and 0.1% NaN₃. The wells were then filled with PBS-BSA-NaN₃ and incubated for a few hours at room temperature.
b) Preparation of peptides and two-site radio immunoassay to determine the inhibitory activity of the analogs;
   (1) All synthetic peptides were dissolved in PBS at a concentration of 10 mM and subsequently diluted serially in PBS-1% BSA/0.1%NaN₃. Twenty-five µl of serial dilutions of each peptide were added to the wells coated with antigen. Control wells received only the diluent. Then, 25 µl of the ¹²⁵I-labelled monoclonal antibody (about 5-10ng, 10⁵cpm) were added to the wells. The microtiter plates were incubated at 4°C for 18 hours, washed 5 times with PBS-BSA-NaN₃ and then the wells were counted for well-bound antibody.

The antibodies (above) and peptides (below) were labeled with ¹²⁵I using Iodogen or Boulton-Hunter reagent, according to the instructions of the manufacturers.

To determine whether the 24-peptide is recognized by all monoclonal antibodies, 5 mg of various types of purified monoclonal antibodies were bound to lml of CNBr activated Sepharose 4B (Pharmacia Fine Chemicals, Piscataway, New Jersey, USA) according to instructions of the manufacturer. The 24 amino acid peptide was radiolabelled with ¹²⁵I with the Boulton-Hunter reagent, to a specific activity of approximately 10⁴ cpm/µg. Twenty µl of beads bearing the different antibodies were incubated for 60 minutes at room temperature in the presence of 100 1 of PBS-BSA containing either 1 µg of the radiolabelled peptide alone or radiolabelled peptide in the presence of 200 µg of cold tetraeicosapeptide. The beads were then washed by centrifugation with PBS-BSA and counted in a gamma-counter.

Table 5 shows that the radiolabelled (1-24) bound specifically to monoclonal antibodies 2G3, 5H8, 8B8 and 8A8 coupled to Sepharose beads. The binding was totally inhibited by adding an excess of cold (1-24) to the incubation mixture. Analogous results were obtained in other experiments using monoclonal antibodies 8E11 and 6B8 (not shown).

### EXAMPLE 11

### Localization of the Epitope within the Tetraeicosapeptide

The inhibiting activity of (1-12) and (1-24) on the binding of the radiolabelled antibodies to the P. knowlesi sporozoite CS protein extracted as described in Example 10 was investigated. The antigen was immobilized on the microtiter wells. Figure 1 demonstrates that for two out of three monoclonal antibodies tested (2G3, 5H8) the epitope must be contained within (1-12), since the inhibitory activities of (1-12)- designated by the white symbols - and (1-24) were almost identical. Similar results were obtained with 8B8 but are not shown in this Figure. By contrast, the binding of antibody 8E11 was inhibited only by (1-24).

### EXAMPLE 12

### Epitope Recognition by Monoclonal Antibodies 2G3, 5H8 and 8B8

The series of shorter analogs and one peptide overlapping two epitopes (7-6) shown in Table VII was used to analyze the specificity of the 2G3, 5H8, and 8B8 antibodies using the inhibition immunoassays described in Examples 10 and 11. As shown in Figs. 7, 8, and 9, and summarized in Table IX, the patterns of reactivity of these antibodies were strikingly similar. The peptides (1-12), (2-12), (3-12), (4-12), (5-12) were strongly inhibitory and as effective as (1-24). On a molar basis, their activities were similar, although in some instances the larger peptides appeared to be slightly better inhibitors. However, a considerable drop in activity was observed in (6-12) and (7-12) as compared to (5-12). These results indicate the presence of an immunodominant epitope around the segment Asp₅-Gly-Ala-Asn-Ala-Gly-Gln-Pro₁₂. To determine the participation of the C-terminal amino acids, the activity of (4-10) and (4-11) was compared to that of (4-12). As also shown in Figs. 7, 8, and 9, the removal of proline (peptide 4-11) had little effect on the reactivity with monoclonal antibodies 2G3 and 8B8, while removal of both glutamine and proline (4-10) diminished markedly the capacity to inhibit the reaction of all three antibodies with the antigen. The shorter peptides (4-9), (5-9), (6-9, (7-9), (8-12), (9-12), and (10-12) were virtually inactive.

### EXAMPLE 13

### Epitope Recognition by Monoclonal Antibody 8E11

As mentioned above, the reaction of antibody 8E11 with the CS protein of P. knowlesi was inhibited by (1-24). About 50% inhibition of binding of radiolabelled 8E11 to the CS protein was observed with a concentration of (1-24) of 10⁻⁵M. In contrast (1-12) had no appreciable inhibitory effect at a concentration of 10⁻²M (See Fig. 6).

### Example 14

The peptide:
Ala₇-Asn₈-Ala₉-Gly₁₀-Gln₁₁-Pro₁₂-Gln₁-Ala₂-Gln₃-Gly₄-Asp₅-Gly₆ was synthesized as described above. Assayed as in the foregoing Examples, this peptide was found to be inactive.

**TABLE I**

| EVIDENCE FOR THE PRESENCE OF AN IMMUNODOMINANT REGION ON THE CS PROTEIN OF P. knowlesi | | | | | | |
|---|---|---|---|---|---|---|
| Cold Inhibitor (Monoclonal Antibody) | % Inhibition of Binding to Antigen of the Radiolabeled Monoclonal Ab | | | | | |
| | 5H8 | 2G3 | 8B8 | 8A8 | 8E11 | 6B8 |
| 5H8 | 100 | 99 | 101 | 115 | 105 | 121 |
| 2G3 | 96 | 100 | 87 | 78 | 106 | 103 |
| 8B8 | 95 | 94 | 100 | 106 | 107 | 125 |
| 8A8 | 86 | 83 | 94 | 100 | 105 | 128 |
| 8E11 | 65 | 59 | 86 | 70 | 100 | 105 |
| 6B8 | 88 | 85 | 91 | 95 | 104 | 100 |
| Control Monoclonal | 6 | 0.4 | 0.5 | 8 | 0 | 0 |
| PBS-BSA | 0 | 0 | 0 | 0 | 0 | 0 |

**TABLE II**

| EVIDENCE FOR THE PRESENCE OF AN IMMUNODOMINANT REGION ON THE CS PROTEIN OF P.vivax | | | | | |
|---|---|---|---|---|---|
| Cold Inhibitor (Monoclonal Antibody) | % Inhibition of Binding to Antigen of the Radiolabeled Monoclonal Ab | | | | |
| | 3D10 | 5D9 | 3C1 | 4E8 | 2F2 |
| 3D10 | 100 | 102 | 99 | 101 | 59 |
| 5D9 | 83 | 100 | 98 | 101 | 65 |
| 3C1 | 113 | 103 | 100 | 102 | 67 |
| 4E8 | 105 | 100 | 99 | 100 | 60 |
| 2F2 | 100 | 101 | 100 | 116 | 100 |
| Control Monoclonal | 8 | -6 | -11 | 3 | 6 |
| PBS-BSA | 0 | 0 | 0 | 0 | 0 |

**TABLE III**

| EVIDENCE FOR THE PRESENCE OF AN IMMUNODOMINANT REGION ON THE CS PROTEIN OF P.falciparum | | | | | |
|---|---|---|---|---|---|
| Cold Inhibitor (Monoclonal Antibody) | % Inhibition of Binding to Antigen of the Radiolabeled Monoclonal Ab | | | | |
| | 3D6 | 2E7 | 1E9 | 2C11 | 2A10 |
| 3D6 | 100 | 98 | 99 | 95 | 75 |
| 2E7 | 98 | 100 | 102 | 99 | 90 |
| 1E9 | 100 | 103 | 100 | 100 | 99 |
| 2C11 | 104 | 100 | 99 | 100 | 100 |
| 2A10 | 110 | 102 | 106 | 100 | 100 |
| Control Monoclonal | 2 | 0 | -4 | 8 | 0 |
| PBS-BSA | 0 | 0 | 0 | 0 | 0 |

**Table IV**

| Results of two-site immunoradiometric assay performed with monoclonal antibody 2G3 and synthetic peptides | | |
|---|---|---|
| Concentration of 12-MER or 24-Mer incubated with the solid-phase antibody 2G3 (µg/ml) | Amount of radiolabeled 2G3 (cpm) bound in wells incubated with: | |
| | 12-MER | 24-MER |
| 500. | 157 | 5517 |
| 50. | 103 | 2056 |
| 5. | 40 | 402 |
| .5 | 0 | 93 |
| .05 | - | 35 |

**Table V**

| A synthetic dodecapeptide (12-MER) inhibits the binding of a monoclonal antibody (2G3) to the CS protein of P. knowlesi | | | | |
|---|---|---|---|---|
| Concentration of P. knowlesi extracts (sporozoites/ml) | Amount of 2G3 bound (cpm) in the presence of the 12-MER inhibitor at concentrations of | | | |
| | 0 | 0.08 | 0.6 | 5.0 |
| | (mg/ml) | | | |
| 10⁶ | 5582 | 3893 | 3018 | 1099 |
| 10⁵ | 4390 | 1221 | 781 | 361 |
| 10⁴ | 1353 | 102 | 70 | 3 |
| 10³ | 156 | 72 | 0 | 37 |
| 0 | 0 | 0 | 0 | 0 |

**Table VI**

| Monoclonal antibody on the Sepharose beads | Inhibitor | % of the radiolabeled 24-MER added which bound to the beads (mean of duplicates) |
|---|---|---|
| 5HB (anti-P. knowlesi) | none | 42.6 |
| | 24-MER | 2.5 |
| 3D11 (anti-P. berghei | none | 6.6 |
| | 24-MER | 2.5 |

**TABLE VII**

| Amino acid composition and preview analysis of synthetic peptide segments of the CS protein P. knowlesi | | | | | | |
|---|---|---|---|---|---|---|
| Peptide Segment | Asp | Pro | Glu | Gly | Ala | Preview |
| 1 - 12 | 1.84(2) | 0.95(1) | 3.00(3) | 3.00(3) | 2.97(3) | 1.4% |
| 2 - 12 | 2.21(2) | 0.85(1) | 2.15(2) | 3.00(3) | 3.02(3) | N.R. |
| 3 - 12 | 2.18(2) | 1.07(1) | 2.00(2) | 2.77(3) | 1.88(2) | N.R. |
| 4 - 12 | 2.00(2) | 1.10(1) | 0.88(1) | 2.84(3) | 1.97(2) | N.R. |
| 5 - 12 | 2.18(2) | 1.17(1) | 1.00(1) | 2.00(2) | 1.93(2) | N.R. |
| 6 - 12 | 0.93(1) | 1.00(1) | 0.97(1) | 2.13(2) | 2.16(2) | N.R. |
| 7 - 12 | 0.90(1) | 1.14(1) | 1.03(1) | 1.00(1) | 2.00(2) | N.R. |
| 8 - 12 | 0.92(1) | 1.05(1) | 1.01(1) | 1.00(1) | 1.00(1) | N.R. |
| 10-12 | -- | 1.10(1) | 1.01(1) | 1.00(1) | -- | N.R. |
| 1 - 24 | 4.15(4) | 1.97(2) | 6.14(6) | 6.05(6) | 5.95(6) | 2.1% |
| 4 - 11 | 2.16(2) | -- | 0.88(1) | 3.40(3) | 2.00(2) | N.R. |
| 5 - 11 | 2.06(2) | -- | 0.95(1) | 2.21(2) | 2.01(2) | N.R. |
| 4 - 10 | 1.88(2) | -- | -- | 3.00(3) | 1.75(2) | N.R. |
| 4 - 9 | 2.00(2) | -- | -- | 2.18(2) | 1.97(2) | N.R. |
| 5 - 9 | 2.00(2) | -- | -- | 0.81(1) | 2.14(2) | N.R. |
| 6 - 9 | 1.00(1) | -- | -- | 0.75(1) | 2.15(2) | N.R. |
| 7 - 9 | 1.00(1) | -- | -- | -- | 2.21(20 | N.R. |
| 7-6* | 1.94(2) | 0.89(1) | 3.06(3) | 2.95(3) | 2.91(3) | N.R. |

| | | | | | | |
|---|---|---|---|---|---|---|
| * peptide bridging two epitopes, i.e. Ala₇Asn₈Ala₉Gly₁₀Gly₁₁Pro₁₂Gln₁Ala₂Gln₃Gly₄Asp₅Gly₆ | | | | | | |

**TABLE VIII**

| Binding of 125I-labelled tetraeicosa-peptide (24 amino acids) to different monoclonal antibodies raised against the sporozoite surface protein of P. knowlesi | | |
|---|---|---|
| Beads bearing the following monoclonal antibodies against the surface protein of P.knowlesi | cpm ( % of input) of radiolabelled tetraeicosa peptide that bound to the beads suspended in: | |
| | PBS-BSA | PBS + excess of cold tetraeisoca peptide |
| 2G3 | 2292 (25.8) | 25 (0.2) |
| 5H8 | 2332 (19.7) | 20 (0.2) |
| 8B8 | 3155 (27.8) | 20 (0.2) |
| 8A8 | 1093 (10.3) | 25 (0.2) |
| Control: beads bearing a monoclonal antibody (3D11) against the surface protein of P.berghei | 25 ( 0.2) | 0 |

**TABLE IX**

| Inhibitory effect of synthetic peptides on the binding of radiolabelled monoclonal antibodies to the CS protein P. knowlesi | | | |
|---|---|---|---|
| Peptide | Molar concentration of peptide (x5) necessary for 50% inhibition of binding of monoclonal antibody | | |
| | 8B8 | 2G3 | 5H8 |
| 1 - 12 | 10⁻¹² | 10⁻¹³ | 10⁻¹² |
| 2 - 12 | 10⁻¹² | 10⁻¹² | 10⁻¹¹ |
| 3 - 12 | 10⁻¹² | 12⁻¹² | 10⁻¹¹ |
| 4 - 12 | 10⁻¹² | 10⁻¹¹ | 10⁻¹² |
| 5 - 12 | 10⁻¹² | 10⁻¹¹ | 10⁻¹¹ |
| 6 - 12 | 10⁻¹⁰ | 10⁻¹⁰ | 10⁻⁹ |
| 7 - 12 | 10⁻⁷ | 10⁻⁶ | 10⁻⁶ |
| 4 - 10 | 10⁻¹⁰ | 10⁻⁵ | 10⁻⁷ |
| 4 - 11 | 10⁻¹² | 10⁻¹² | 10⁻¹⁰ |

## Claims (Claims for the following Contracting State(s): BE, CH, LI, DE, FR, GB, LU, NL, SE)

1. A peptide containing at least one occurence of an amino acid sequence corresponding to a repeating unit of the immunodominant epitope region of the circumsporozoite protein of a member of the genus Plasmodium, said region containing tandem occurences of said unit and said peptide comprising all or part of said region, but not being the entire circumsporozoite protein, wherein said unit contains up to twelve amino acids and said peptide elicits species specific antibodies recognizing the circumsporozoite protein upon injection of said peptide into a mammal.

2. The peptide according to claim 1, wherein the member of the genus Plasmodium is selected from the group comprising P. falciparum, P. vivax, P. knowlesi, P. malariae, P. cynomolgi, P. berghei and P. yoeli nigeriensis.

3. The peptide according to any of claims 1 or 2, wherein the sequence of said peptide is Gln Ala Gln Gly Asp Gly Ala Asn Ala Gly Gln Pro.

4. The peptide according to any of claims 1 to 3, wherein the sequence of said peptide is Asp-Gly-Ala-Asn-Ala-Gly-Gln-Pro.

5. The peptide according to any of claims 1 to 3, wherein the sequence of said peptide is Gly-Asp-Gly-Ala-Asn-Ala-Gly-Gln.

6. The peptide according to any of claims 1 to 5, said peptide being immunochemically reactive with at least two monoclonal antibodies to said circumsporozoite protein.

7. The peptide according to any of claims 1 to 6, wherein said peptide confers protective immunity to a host against the sporozoite stage of malaria.

8. The peptide according to any of claims 1 to 7 fused to a portion of a procaryotic protein.

9. The peptide according to any of claims 1 to 7 chemically synthesized.

10. The peptide according to any of claims 1 to 8 synthesized by expression of a recombinant microorganism.

11. A vaccine against malaria comprising as an active ingredient the peptide of any of claims 1 to 10 and a carrier.

12. The vaccine of claim 11 wherein said peptide is adsorbed or covalently attached to a carrier protein.

13. The vaccine of any of claims 11 or 12 comprising a peptide according to any of claims 1 to 10 in a physiologically acceptable medium.

14. The vaccine of claim 14 wherein said peptide is immunochemically reactive with a monoclonal or polyclonal antibody to a sporozoite CS protein of the genus Plasmodium.

15. A DNA strand coding for the amino acid sequence of the peptide according to any of claims 1 to 10.

16. A DNA transfer vector comprising an inserted DNA strand said strand comprising as an essential element a deoxynucleotide sequence according to claim 15.

17. A process for the production of a polypeptide according to any of claims 1 to 7, characterized in that the polypeptide is produced by sequential addition of amino acids using conventional peptide synthesis methods based on the sequence information derived by screening a cDNA library for an expressed protein containing the immunochemically reactive region of the sporozoite surface antigen protein using a monoclonal antibody and determining the amino acid sequence of the repeat epitope of the said immunochemically reactive region.

18. A process for the production of a polypeptide according to any of claims 1 to 8 characterized in that the polypeptide is produced by culturing a microorganism containing a recombinant vector and recovering the polypeptide from the culture, wherein said vector is obtainable by screening a cDNA library for an expressed protein containing the immunochemically reactive region of the sporozoite surface antigen protein using a monoclonal antibody and inserting the Plasmodium specific DNA encoding the repeat epitope of the immunodominant region of a circumsporozoite protein into the vector, said vector being capable of expressing the polypeptide.

## Claims (Claims for the following Contracting State(s): AT)

1. A process for the production of a polypeptide containing at least one occurence of an amino acid sequence corresponding to a repeating unit of the immunodominant epitope region of the circumsporozoite protein of a member of the genus Plasmodium, said region containing tandem occurences of said unit and said peptide comprising all or part of said region, but not being the entire circumsporozoite protein, wherein said unit contains up to twelve amino acids and said peptide elicits species specific antibodies recognizing the circumsporozoite protein upon injection of said peptide into a mammal,characterized in that the polypeptide is produced by sequential addition of amino acids using conventional peptide synthesis methods based on the sequence information derived by screening a cDNA library for an expressed protein containing the immunochemically reactive region of the sporozoite surface antigen protein using a monoclonal antibody and determining the amino acid sequence of the repeat epitope of the said immunochemically reactive region.

2. A process for the production of a polypeptide containing at least one occurence of an amino acid sequence corresponding to a repeating unit of the immunodominant epitope region of the circumsporozoite protein of a member of the genus Plasmodium, said region containing tandem occurences of said unit and said peptide comprising all or part of said region, but not being the entire circumsporozoite protein, wherein said unit contains up to twelve amino acids and said peptide elicits species specific antibodies recognizing the circumsporozoite protein upon injection of said peptide into a mammal, characterized in that the polypeptide is produced by culturing a microorganism containing a recombinant vector and recovering the polypeptide from the culture, wherein said vector is obtainable by screening a cDNA library for an expressed protein containing the immunochemically reactive region of the sporozoite surface antigen protein using a monoclonal antibody and inserting the Plasmodium specific DNA encoding the repeat epitope of the immunodominant region of a circumsporozoite protein into the vector, said vector being capable of expressing the polypeptide.

3. The process according to any of claims 1 or 2, wherein the member of the genus Plasmodium is selected from the group comprising P. falcinarum, P. vivax, P. knowlesi, P. malariae, P. cynomolgi, P. berghei and P. yoeli nigeriensis.

4. The process according to any of claims 1 to 3, wherein the sequence of said peptide is Gln Ala Gln Gly Asp Gly Ala Asn Ala Gly Gln Pro.

5. The process according to any of claims 1 to 4, wherein the sequence of said peptide is Asp-Gly-Ala-Asn-Ala-Gly-Gln-Pro.

6. The process according to any of claims 1 to 5, wherein the sequence of said peptide is Gly-Asp-Gly-Ala-Asn-Ala-Gly-Gln.

7. The process according to any of claims 1 to 6, said peptide being immunochemically reactive with at least two monoclonal antibodies to said circumsporozoite protein.

8. The process according to any of claims 1 to 7, wherein said peptide confers protective immunity to a host against the sporozoite stage of malaria.

9. The process according to any of claims 1 to 8 fused to a portion of a procaryotic protein.

10. A method for preparing a vaccine against malaria comprising combining as an active ingredient the peptide producible according to any of claims 1 to 9 and a carrier.

11. The method according to claim 10 wherein said peptide is adsorbed or covalently attached to a carrier protein.

12. The method according to any of claims 10 or 11 comprising combining a peptide producible according to any of claims 1 to 9 and a physiologically acceptable medium.

13. The method according to claim 12 wherein said peptide is immunochemically reactive with a monoclonal or polyclonal antibody to a sporozoite CS protein of the genus Plasmodium.

14. A method for producing a DNA strand coding for the amino acid sequence of the peptide producible according to any of claims 1 to 9, wherein the DNA strand is chemically synthesized or is obtained from a recombinant microorganism.

15. A method for producing a DNA transfer vector comprising inserting a DNA strand into a cloning vector, said strand comprising as an essential element a deoxynucleotide sequence produced according to claim 14.

## Patentansprüche (Patentansprüche für folgende(n) Vertragsstaat(en): BE, CH, LI, DE, FR, GB, LU, NL, SE)

1. Peptid, enthaltend mindestens ein Vorkommen einer Aminosäuresequenz, die einer wiederholten Einheit des Bereiches des immundominanten Epitopes des Circumsporozoitenproteins eines Mitglieds der Gattung Plasmodium entspricht, wobei dieser Bereich tandemartige Vorkommen der Einheit enthält und das Peptid den gesamten Bereich oder einen Teil des Bereiches umfaßt, aber nicht dem gesamten Circumsporozoitenprotein entspricht, wobei diese Einheit bis zu 12 Aminosäuren enthält und das Peptid Art-spezifische Antikörper hervorruft, die das Circumsporozoitenprotein nach Injektion des Peptides in ein Säugetier erkennen.

2. Peptid nach Anspruch 1, wobei das Mitglied der Gattung Plasmodium aus der P. falciparum, P. vivax, P. knowlesi, P. malariae, P. cynomolgi, P. berghei und P. yoeli nigeriensis umfassenden Gruppe ausgewählt ist.

3. Peptid nach einem der Ansprüche 1 oder 2, wobei die Sequenz des Peptides Gln Ala Gln Gly Asp Gly Ala Asn Ala Gly Gln Pro ist.

4. Peptid nach einem der Ansprüche 1 bis 3, wobei die Sequenz des Peptides Asp-Gly-Ala-Asn-Ala-Gly-Gln-Pro ist.

5. Peptid nach einem der Ansprüche 1 bis 3, wobei die Sequenz des Peptides Gly-Asp-Gly-Ala-Asn-Ala-Gly-Gln ist.

6. Peptid nach einem der Ansprüche 1 bis 5, wobei das Peptid mit mindestens zwei monoklonalen Antikörpern gegen das Circumsporozoitenprotein immunchemisch reaktiv ist.

7. Peptid nach einem der Ansprüche 1 bis 6, wobei das Peptid einem Wirt schützende Immunität gegen das Sporozoitenstadium von Malaria verleiht.

8. Peptid nach einem der Ansprüche 1 bis 7, wobei das Peptid an einen Teil eines prokaryontischen Proteins fusioniert ist.

9. Peptid nach einem der Ansprüche 1 bis 7, das chemisch synthetisiert ist.

10. Peptid nach einem der Ansprüche 1 bis 8, das durch Expression eines rekombinanten Mikroorganismus synthetisiert ist.

11. Impfstoff gegen Malaria, umfassend das Peptid eines der Ansprüche 1 bis 10 als einen aktiven Bestandteil und einen Träger.

12. Impfstoff nach Anspruch 11, wobei das Peptid an ein Trägerprotein adsorbiert oder kovalent angeheftet ist.

13. Impfstoff nach einem der Ansprüche 11 oder 12, umfassend ein Peptid nach einem der Ansprüche 1 bis 10 in einem physiologisch verträglichen Medium.

14. Impfstoff nach Anspruch 13, wobei das Peptid mit einem monoklonalen oder polyklonalen Antikörper gegen ein Sporozoiten-CS-Protein der Gattung Plasmodium immunchemisch reaktiv ist.

15. DNA-Strang, der für die Aminosäuresequenz des Peptides gemäß einem der Ansprüche 1 bis 10 codiert.

16. DNA-Transfervektor, umfassend einen insertierten DNA-Strang, wobei der Strang als ein wesentliches Element eine Desoxynukleotidsequenz gemäß Anspruch 15 umfaßt.

17. Verfahren zum Herstellen eines Polypeptides nach einem der Ansprüche 1 bis 7, **dadurch gekennzeichnet**, daß das Polypeptid durch aufeinanderfolgende Addition von Aminosäuren unter Verwendung konventioneller Peptidsyntheseverfahren auf der Grundlage von Sequenzinformation erzeugt wird, die durch das Durchmustern einer cDNA-Bank unter Verwendung eines monoklonalert Antikörpers auf ein exprimiertes Protein, das den immunchemisch reaktiven Bereich des Sporozoiten-Oberflächenantigenproteins enthält, und Bestimmen der Aminosäuresequenz des wiederholten Epitops des immunchemisch reaktiven Bereiches abgeleitet wird.

18. Verfahren zum Erzeugen eines Polypeptides nach einem der Ansprüche 1 bis 8, **dadurch gekennzeichnet**, daß das Polypeptid durch Kultivieren eines Mikroorganismus, der einen rekombinanten Vektor enthält, und Gewinnen des Polypeptides aus der Kultur erzeugt wird, wobei der Vektor durch Durchmustern einer cDNA-Bank auf ein exprimiertes Protein, das den immunchemisch reaktiven Bereich des Sporozoiten-Oberflächenantigenproteins enthält, unter Verwendung eines monoklonalen Antikörpers und Insertieren der Plasmodium-spezifischen DNA, die für das wiederholte Epitop des immundominanten Bereiches eines Circumsporozoitenproteins in dem Vektor codiert, erhältlich ist, wobei der Vektor zur Expression des Polypeptides fähig ist.

## Patentansprüche (Patentansprüche für folgende(n) Vertragsstaat(en): AT)

1. Verfahren zum Erzeugen eines Polypeptides, das mindestens ein Vorkommen einer Aminosäuresequenz enthält, die einer wiederholten Einheit des Bereiches des immundominanten Epitopes eines Circumsporozoitenproteins eines Mitglieds der Gattung Plasmodium entspricht, wobei der Bereich tandemartige Vorkommen der Einheit enthält, und das Peptid den gesamten Bereich oder einen Teil des Bereiches umfaßt, aber nicht dem gesamten Circumsporozoitenprotein entspricht, wobei die Einheit bis zu 12 Aminosäuren enthält und das Peptid Art-spezifische Antikörper hervorruft, die das Circumsporozoitenprotein nach Injektion des Peptides in ein Säugetier erkennen, **dadurch gekennzeichnet**, daß das Polypeptid durch aufeinanderfolgende Addition von Aminosäuren unter Verwendung konventioneller Peptidsyntheseverfahren auf der Grundlage von Sequenzinformationen erzeugt wird, die durch das Durchmustern einer cDNA-Bank unter Verwendung eines monoklonalen Antikörpers auf ein exprimiertes Protein, das den immunchemisch reaktiven Bereich des Sporozoiten-Oberflächen antigenproteins enthält, und Bestimmen der Aminosäuresequenz des wiederholten Epitops des immunchemisch reaktiven Bereiches abgeleitet wird.

2. Verfahren zum Erzeugen eines Polypeptides, das mindestens ein Vorkommen einer Aminosäuresequenz enthält, die einer wiederholten Einheit des Bereiches des immundominanten Epitopes des Circumsporozoitenproteins eines Mitgliedes der Gattung Plasmodium entspricht, wobei der Bereich tandemartige Vorkommen der Einheit enthält, und das Peptid den ganzen Bereich oder einen Teil des Bereiches umfaßt, aber nicht dem gesamten Circumsporozoitenprotein entspricht, wobei die Einheit bis zu 12 Aminosäuren enthält und das Peptid Art-spezifische Antikörper hervorruft, die das Circumsporozoitenprotein nach Injektion des Peptides in ein Säugetier erkennen, **dadurch gekennzeichnet**, daß das Polypeptid durch Kultivieren eines Mikroorganismus, der einen rekombinanten Vektor enthält, und Gewinnen des Polypeptides aus der Kultur erzeugt wird, wobei der Vektor durch Durchmustern einer cDNA-Bank auf ein exprimiertes Protein, das den immunchemisch reaktiven Bereich des Sporozoiten-Oberflächenantigenproteins enthält, unter Verwendung eines monoklonalen Antikörpers und Insertieren der Plasmodium-spezifischen DNA, die für das wiederholte Epitop des immundominanten Bereiches eines Circumsporozoitenproteins in dem Vektor codiert, erhältlich ist, wobei der Vektor zur Expression des Polypeptides fähig ist.

3. Verfahren nach einem der Ansprüche 1 oder 2, wobei das Mitglied der Gattung Plasmodium P. falciparum, P. vivax, P. knowlesi, P. malariae, P. cynomolgi, P. berghei und P. yoeli nigeriensis umfassenden Gruppe ausgewählt ist.

4. Verfahren nach einem der Ansprüche 1 bis 3, wobei die Sequenz des Peptides Gln Ala Gln Gly Asp Gly Ala Asn Ala Gly Gln Pro ist.

5. Verfahren nach einem der Ansprüche 1 bis 4, wobei die Sequenz des Peptides Asp-Gly-Ala-Asn-Ala-Gly-Gln-Pro ist.

6. Verfahren nach einem der Ansprüche 1 bis 5, wobei die Sequenz des Peptides Gly-Asp-Gly-Ala-Asn-Ala-Gly-Gln ist.

7. Verfahren nach einem der Ansprüche 1 bis 6, wobei das Peptid mit mindestens zwei monoklonalen Antikörpern gegen das Circumsporozoitenprotein immunchemisch reaktiv ist.

8. Verfahren nach einem der Ansprüche 1 bis 7, wobei das Peptid einem Wirt schützende Immunität gegen das Sporozoitenstadium von Malaria verleiht.

9. Verfahren nach einem der Ansprüche 1 bis 8, wobei das Protein an einen Teil eines prokaryontischen Proteins fusioniert ist.

10. Verfahren zum Herstellen eines Impfstoffes gegen Malaria, umfassend das Kombinieren des Peptides, herstellbar gemäß einem der Ansprüche 1 bis 9, als einem aktiven Bestandteil mit einem Träger.

11. Verfahren nach Anspruch 10, wobei das Peptid an ein Trägerprotein adsorbiert oder kovalent angeheftet ist.

12. Verfahren nach einem der Ansprüche 10 oder 11, umfassend das Kombinieren eines Peptides, das gemäß einem der Ansprüche 1 bis 9 herstellbar ist, und eines physiologisch verträglichen Mediums.

13. Verfahren nach Anspruch 12, wobei das Peptid mit einem monoklonalen oder polyklonalen Antikörper gegen ein Sporozoiten-CS-Protein der Gattung Plasmodium immunchemisch reaktiv ist.

14. Verfahren zum Erzeugen eines DNA-Stranges, der für eine Aminosäuresequenz des Peptides codiert, das nach einem der Ansprüche 1 bis 9 herstellbar ist, wobei der DNA-Strang chemisch synthetisiert wird oder aus einem rekombinanten Mikroorganismus erhalten wird.

15. Verfahren zum Erzeugen eines DNA-Transfervektors, umfassend das Insertieren eines DNA-Stranges in einen Clonierungsvektor, wobei der Strang als ein wesentliches Element eine gemäß Anspruch 14 hergestellte Desoxynukleotidsequenz enthält.

## Revendications (Revendications pour l'(les) Etat(s) contractant(s) suivant(s): BE, CH, LI, DE, FR, GB, LU, NL, SE)

1. Peptide contenant au moins une survenance d'une séquence d'amino acides correspondant à une unité de répétition de la région d'épitope immunodominante de la protéine de circumsporozoïte d'un membre du genre Plasmodium, ladite région contenant des survenances en tandem de ladite unité et ledit peptide comprenant tout ou partie de ladite région, mais n'étant pas la protéine de circumsporozoïte entière, dans lequel ladite unité contient jusqu'à douze amino acides et ledit peptide suscite des anticorps spécifiques de l'espèce, reconnaissant la protéine de circumsporozoïte lors de l'injection dudit peptide à un mammifère.

2. Peptide selon la revendication 1, dans lequel le membre du genre Plasmodium est choisi dans le groupe comprenant P. falciparum, P. vivax, P. knowlesi, P. malariae, P. cynomolgi, P. berghei et P. yoeli nigeriensis.

3. Peptide selon l'une quelconque des revendications 1 ou 2, dans lequel la séquence dudit peptide est Gln Ala Gln Gly Asp Gly Ala Asn Ala Gly Gln Pro.

4. Peptide selon l'une quelconque des revendications 1 à 3, dans lequel la séquence dudit peptide est Asp-Gly-Ala-Asn-Ala-Gly-Gln-Pro.

5. Peptide selon l'une quelconque des revendications 1 à 3, dans lequel la séquence dudit peptide est Gly-Asp-Gly-Ala-Asn-Ala-Gly-Gln.

6. Peptide selon l'une quelconque des revendications 1 à 5, ledit peptide étant immunochimiquement réactif avec au moins deux anticorps monoclonaux contre ladite protéine de circumsporozoïte.

7. Peptide selon l'une quelconque des revendications 1 à 6, dans lequel ledit peptide confère une immunité protectrice à un hôte contre le stade sporozoïte de la malaria.

8. Peptide selon l'une quelconque des revendications 1 à 7 fusionné à une portion d'une protéine procaryotique.

9. Peptide selon l'une quelconque des revendications 1 à 7 préparé par synthèse chimique.

10. Peptide selon l'une quelconque des revendications 1 à 8 synthétisé par expression d'un microorganisme recombinant.

11. Vaccin contre la malaria comprenant comme ingrédient actif le peptide selon l'une quelconque des revendications 1 à 10 et un support.

12. Vaccin selon la revendication 11, dans lequel ldit peptide est adsorbé ou lié par covalence à une protéine de support.

13. Vaccin selon l'une quelconque des revendications 11 ou 12 comprenant un peptide selon l'une quelconque des revendications 1 à 10 dans un milieu physiologiquement acceptable.

14. Vaccin selon la revendication 13, dans lequel ledit peptide est immunochimiquement réactif avec un anticorps monoclonal ou polyclonal contre une protéine CS de sporozoïte du genre Plasmodium.

15. Brin d'ADN codant pour la séquence d'amino acides du peptide selon l'une quelconque des revendications 1 à 10.

16. Vecteur de transfert d'ADN comprenant un brin d'ADN inséré, ledit brin comprenant comme élément essentiel une séquence de désoxynucléotide selon la revendication 15.

17. Procédé pour la production d'un polypeptide selon l'une quelconque des revendications 1 à 7, caractérisé en ce que le polypeptide est produit par addition séquentielle d'amino acides en utilisant des procédés classiques de synthèse du peptide basés sur l'information sur la séquence obtenue par triage d'une bibliothèque d'ADNc pour une protéine exprimée contenant la région immunochimiquement réactive de la protéine d'antigène de surface du sporozoïte en utilisant un anticorps monoclonal et en déterminant la séquence d'amino acides de l'épitope de répétition de ladite région immunochimiquement réactive.

18. Procédé pour la production d'un polypeptide selon l'une quelconque des revendications 1 à 8, caractérisé en ce que le polypeptide est produit par culture d'un microorganisme contenant un vecteur recombinant et en récupérant le polypeptide à partir de la culture, dans lequel ledit vecteur peut être obtenu par triage d'une bibliothèque d'ADNc pour une protéine exprimée contenant la région immunochimiquement réactive de la protéine d'antigène de surface du sporozoïte en utilisant un anticorps monoclonal et en insérant l'ADN spécifique du Plasmodium codant pour l'épitope de répétition de la région immunodominante d'une protéine de circumsporozoïte dans le vecteur, ledit vecteur étant capable d'exprimer le polypeptide.

## Revendications (Revendications pour l'(les) Etat(s) contractant(s) suivant(s): AT)

1. Procédé pour la production d'un polypeptide contenant au moins une survenance d'une séquence d'amino acides correspondant à une unité de répétition de la région d'épitope immunodominante de la protéine de circumsporozoïte d'un membre du genre Plasmodium, ladite région contenant des survenances en tandem de ladite unité et ledit peptide comprenant toute ou partie de ladite région, mais n'étant pas la protéine de circumsporozoïte entière, dans lequel ladite unité contient jusqu'à douze aminoacides et ledit peptide suscite des anticorps spécifiques de l'espèce, reconnaissant la protéine de circumsporozoïte lors de l'injection dudit peptide à un mammifère, caractérisé en ce que le polypeptide est produit par addition séquentielle d'amino acides en utilisant des procédés classiques de synthèse du peptide basés sur l'information sur la séquence obtenue par triage d'une bibliothèqe d'ADNc pour une protéine exprimée contenant la région immunochimiquement réactive de la protéine d'antigène de surface du sporozoïte en utilisant un anticorps monoclonal et en déterminant la séquence d'amino acides de l'épitope de répétition de ladite région immunochimiquement réactive.

2. Procédé pour la production d'un polypeptide contenant au moins une survenance d'une séquence d'amino acides correspondant à une unité de répétition de la région d'épitope immunodominante de la protéine de circumsporozoïte d'un membre du genre Plasmodium, ladite région contenant des survenances en tandem de ladite unité et ledit peptide comprenant tout ou partie de ladite région, mais n'étant pas la protéine de circumsporozoïte entière, dans lequel ladite unité contient jusqu'à douze aminoacides et ledit peptide suscite des anticorps spécifiques de l'espèce, reconnaissant la protéine de circumsporozoïte lors de l'injection dudit peptide à un mammifère, caractérisé en ce que le polypeptide est produit par culture d'un microorganisme contenant un vecteur recombinant et récupération du polypeptide à partir de la culture, dans lequel ledit vecteur peut être obtenu par triage d'une bibliothèque d'ADNc pour une protéine exprimée contenant la région immunochimiquement réactive de la protéine d'antigène de surface du sporozoïte en utilisant un anticorps monoclonal et en insérant l'ADN spécifique du Plasmodium codant pour l'épitope de répétition de la région immunodominante d'une protéine de circumsporozoïte dans le vecteur, ledit vecteur étant capable d'exprimer le polypeptide.

3. Procédé selon l'une quelconque des revendications 1 ou 2, dans lequel le membre du genre Plasmodium est choisi dans le groupe comprenant P. falciparum, P. vivax, P. knowlesi, P. malariae, P. cynomolgi, P. berghei et P. yoeli nigeriensis.

4. Procédé selon l'une quelconque des revendications 1 à 3, dans lequel la séquence dudit peptide est Gln Ala Gln Gly Asp Gly Ala Asn Ala Gly Gln Pro.

5. Procédé selon l'une quelconque des revendications 1 à 4, dans lequel la séquence dudit peptide est Asp-Gly-Ala-Asn-Ala-Gly-Gln-Pro.

6. Procédé selon l'une quelconque des revendications 1 à 5, dans lequel la séquence dudit peptide est Gly-Asp-Gly-Ala-Asn-Ala-Gly-Gln.

7. Procédé selon l'une quelconque des revendications 1 à 6, ledit peptide étant immunochimiquement réactif avec au moins deux anticorps monoclonaux contre ladite protéine de circumsporozoïte.

8. Procédé selon l'une quelconque des revendications 1 à 7, dans lequel ledit peptide confère une immunité protectrice à un hôte contre le stade sporozoïte de la malaria.

9. Procédé selon l'une quelconque des revendications 1 à 8, dans lequel ledit peptide est fusionné à une portion d'une protéine procaryotique.

10. Procédé de préparation d'un vaccin contre la malaria comprenant le fait de combiner comme ingrédient actif le peptide pouvant être produit selon l'une quelconque des revendications 1 à 9 et un support.

11. Procédé selon la revendication 10, dans lequel ledit peptide est adsorbé ou lié par covalence à une protéine de support.

12. Procédé selon l'une quelconque des revendications 10 ou 11, comprenant le fait de combiner un peptide pouvant être produit selon l'une quelconque des revendications 1 à 9 et un milieu physiologiquement acceptable.

13. Procédé selon la revendication 12, dans lequel ledit peptide est immunochimiquement réactif avec un anticorps monoclonal ou polyclonal contre une protéine CS de sporozoite du genre Plasmodium.

14. Procédé de préparation d'un brin d'ADN codant pour la séquence d'amino acides du peptide pouvant être produit selon l'une quelconque des revendications 1 à 9, dans lequel le brin d'ADN est préparé par synthèse chimique ou est obtenu à partir d'un microorganisme recombinant.

15. Procédé pour la préparation d'un vecteur de transfert d'ADN comprenant l'insertion d'un brin d'ADN dans un vecteur de clonage, ledit brin comprenant comme élément essentiel une séquence de désoxynucléotide produite selon la revendication 14.
